(19) Europäisches Patentamt
European Patent Office
Office européen des brevets

(11) EP 1 728 504 B1

(12) # EUROPEAN PATENT SPECIFICATION

(45) Date of publication and mention of the grant of the patent:
**31.07.2013 Bulletin 2013/31**

(21) Application number: **05727183.5**

(22) Date of filing: **23.03.2005**

(51) Int Cl.:
*A61K 9/06* (2006.01) *A61K 9/08* (2006.01)
*A61K 9/107* (2006.01) *A61K 9/48* (2006.01)

(86) International application number:
**PCT/JP2005/005238**

(87) International publication number:
**WO 2005/089714 (29.09.2005 Gazette 2005/39)**

(54) **EMULSION STABILIZER**

EMULSIONSSTABILISATOR

STABILISATEUR D'EMULSION

(84) Designated Contracting States:
**AT BE BG CH CY CZ DE DK EE ES FI FR GB GR HU IE IS IT LI LT LU MC NL PL PT RO SE SI SK TR**

(30) Priority: **24.03.2004 JP 2004087023**

(43) Date of publication of application:
**06.12.2006 Bulletin 2006/49**

(73) Proprietor: **Takeda Pharmaceutical Company Limited**
**Osaka-shi,**
**Osaka 541-0045 (JP)**

(72) Inventor: **YOSHINARI, Tomohiro**
**Osaka-shi,**
**Osaka 532-8686 (JP)**

(74) Representative: **von Kreisler Selting Werner**
**Deichmannhaus am Dom**
**Bahnhofsvorplatz 1**
**50667 Köln (DE)**

(56) References cited:
**EP-A- 1 236 476**
**WO-A-03/055466**
**WO-A1-03/055466**
**JP-A- 2 121 929**
**JP-A- 8 073 476**
**JP-A- 8 277 215**
**JP-A- 60 208 927**
**JP-A- 2002 522 379**
**JP-A- 2003 335 776**
**US-A- 6 054 136**
**US-A- 6 054 136**
**US-B2- 6 652 865**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 728 504 B1

Printed by Jouve, 75001 PARIS (FR)

## Description

Technical Field

The present invention relates to an oral semisolid or liquid pharmaceutical composition that forms or maintains a stable emulsion, and to a preparation that comprises this composition.

Background Art

The Self Micro-Emulsifying Drug Delivery System (SMEDDS™) is a drug delivery system developed by Gattefossé (France). This system is composed of three components, a surfactant, a cosurfactant, and a lipid phase (refer to patent document 1 below). The present system is a compatible mixture of these three components, which is characterized in that a microemulsion is spontaneously produced when water is added thereto as a fourth component. A well-known example of an application of this technology is Neoral™, which is a Cyclosprin immunosuppressive agent preparation that is commercially available from Novartis.

On the other hand, digestive juices are different from pure water insofar as they contain various types of ion species. In addition, the pH value is different depending on the location in the digestive tract. For this reason, cases are not unusual in which a microemulsion is formed in pure water but is not formed in the digestive tract. Thus, a system is desired in which a microemulsion is formed or maintained with favorable reproducibility even in the digestive tract, in which there are many factors that act adversely on emulsion stability.

US 6 054 136 discloses a liquid oral micro-emulsion for oral administration comprising a lipophilic phase, a surfactant, a co-surfactant and a pharmaceutical active ingredient. For example, example 6 shows a mix for filling capsules comprising $C_8$-$C_{18}$ poly-glycolized fatty acids glycerides, $C_8$-$C_{10}$ poly-glycolized fatty acids glycerides and an active ingredient.

WO 03/055466 discloses a liquid pharmaceutical micro-emulsion for capsules, comprising an active ingredient, a polyoxyethylene-hydrogenated castor oil (cremophor RH 40) as first surfactant and as second surfactant polyethylene glycol-caprylic acid/capric acid.

EP 1 236 476 discloses a pharmaceutical composition for oral use, wherein an emulsion is formed by dissolving or dispersing (1) a water-insoluble or slightly soluble medicinal compound represented by the same formula as claimed in claim 12, in (2) a carrier comprising two or more surfactants having different HLB. The examples use a combination of saturated and unsaturated polyglycolized glycerides such as Labrasol (halving a high HLB *ie*.$^3$8) and Labrafil WL (having a low HLB). The polyoxyethylene-hydrogenated castor oil, Cremophor EL, is foreseen as another suitable surfactant.

Disclosure of Invention

Problems to be Solved by the Invention

The present invention has the objective of providing a pharmaceutical composition that forms a stable microemulsion in the digestive tract which includes a medicinal component, specifically, a water-insoluble medicinal component, and has a high bioavailability; and a manufacturing method thereof.

Means of Solving the Problems

The present invention provides:

(1) A semisolid or liquid oral pharmaceutical composition comprising a medicinal compound and two or more surfactants having different molecular weights as disclosed in the claims.

Effects of the Invention

The pharmaceutical composition of the present invention forms a stable microemulsion, or can maintain this stable microemulsion. In addition, when a preparation containing the pharmaceutical composition of the present invention is administered orally, a stable microemulsion in which microparticles comprising an active ingredient are dispersed is formed or maintained in the digestive tract, and as a result, the absorbability of the active ingredient, particularly a hardly water-soluble active ingredient from the digestive tract is greatly improved, and the bioavailability thereof becomes higher.

Brief Description of Drawings

Fig. 1 is a Photograph comparing the liquid condition of compositions obtained in Example 1 and Reference Example 1.
Fig. 2 is a Diagram showing the results of turbidity evaluation (absorption ratio at a wavelength of 550 nm) for the compositions obtained in Example 1 and Reference Example 1.
Fig. 3 is a Photograph comparing the liquid state after elution test for the compositions obtained in Example 6 and Reference Example 2.
Fig. 4 is a Graph comparing the change in blood concentration of compound A for oral administration of the compositions obtained in Example 1 and Reference Example 1.

Explanation of Symbols

(A) Purified water
(B) 1 M NaCl aqueous solution
(C) Japanese Pharmacopoeia No. 2 solution (pH 6.8)

Best Mode for Carrying Out the Invention

The oral pharmaceutical composition of the present invention is a semisolid or liquid composition which comprises two or more surfactants having different molecular weights and which has the function to uniformly disperse an active ingredient into a microemulsion while in the digestive tract. In the present specification, the term "microemulsion" denotes a material in which a disperse phase (active ingredient-containing phase) is in a state of being solubilized in a disperse medium via surfactant micelles, and the droplet diameter is 400 nm or less, preferably 100 nm or less, and more preferably 50 nm or less. The microemulsion is a system that is thermodynamically stable, and is substantially different from a common emulsion (macroemulsion) that is thermodynamically unstable. In addition, the droplet diameter of microemulsion can be measured with any suitable method of laser scattering method (Mie theory) and dynamic light scattering method (photon correlation method). For example, measurement can be carried out using LA-920 (manufactured by Horiba Ltd.) in case of laser scattering method, and LB-550 (manufactured by Horiba Ltd.) in case of dynamic light scattering method. The semisolid or liquid pharmaceutical composition of the present invention may be transparent or not transparent.

Further, forming an emulsion by the pharmaceutical composition of the present invention can be easily evaluated by measuring turbidity of dispersion liquid wherein 0.3 g of the pharmaceutical composition is dispersed in 20 mL of solvent (1M sodium chloride aqueous solution or Japanese Pharmacopoeia No. 2 solution) which is warmed to 40°C, using spectrophotometer (wave length 550 nm, cell length 1 cm). Then, the absorbance is 0.2 or less, preferably 0.1 or less, and more preferably 0.05 or less for either solvent to be used.

The pharmaceutical composition for oral use of the present invention contains 2 or more surfactants having different molecular weights. Here, the difference of molecular weights between surfactant having minimum molecular weight and surfactant having maximum molecular weight is preferably 300 or more, more preferably 800 or more, and most preferably 1,200 or more.

In the present invention, the two or more surfactants having different molecular weights are preferably surfactants of a homologous series. The term "homologous series" in the "surfactants of a homologous series" used herein means that the relevant surfactants are each composed of same kind of constituent unit such as the relationship between long-chain fatty acid glycerides having long-chain polyoxyethylenes as hydrophilic groups, and medium-chain fatty acid glycerides having short-chain polyoxyethylenes as hydrophilic groups.

Nonionic surfactants and surfactants derived from natural materials and the like may be used as the above surfactants. Examples of the above nonionic surfactants to be used include glycerin fatty acid esters, fatty acid-ethylene oxide adducts, higher alcohol-ethylene oxide adducts, alkylphenol-ethylene oxide adducts, polyhydric alcohol fatty acid ester-ethylene oxide adducts, higher alkylamine-ethylene oxide adducts, fatty acid amide-ethylene oxide adducts, oil-ethylene oxide adducts, pentaerythritol fatty acid esters, polyhydric alcohol alkyl ethers, fatty acid amides of alkanolamines, and the like. Specifically, sorbitol and sorbitan fatty acid esters, polyoxyethylene sorbitan fatty acid esters, polyethylene glycol fatty acid esters, sucrose fatty acid esters, polyoxyethylenated glycerin fatty acid esters, polyoxyethylene castor oil (polyethoxylated castor oil), polyoxyethylene-hydrogenated castor oil (polyethoxylated hydrogenated castor oil), polyoxyethylene polypropylene glycol copolymer, glycerin fatty acid ester, polyglycerin fatty acid ester, and

the like are preferably used.

Examples of the natural-derived surfactants to be used include lecithin phospholipids such as egg yolk lecithin (trade name: PL-100H, QP corporation), soy lecithin (trade name: Lecinol S-10, Nikko Chemicals), and the like.

In addition, the two or more surfactants having different molecular weights in the present invention is preferred to have HLB value of 12 or more, and preferably have HLB value of 14 or more.

Fatty acid glycerides having polyoxyethylene chains as hydrophilic groups are preferred as the two or more surfactants with different molecular weights in the present invention; specifically, combinations of long-chain fatty acid glycerides having long-chain polyoxyethylenes as hydrophilic groups, and medium-chain fatty acid glycerides having short-chain polyoxyethylenes as hydrophilic groups are preferred. More specifically, exemplified a combination in which the long-chain fatty acid glycerides having long-chain polyoxyethylenes as hydrophilic groups are $C_{14-20}$ fatty acid glycerides having polyoxyethylene chains in which the number of repetitions of ethylene oxide unit is 20 to 500 as hydrophilic groups and the medium-chain fatty acid glycerides having short-chain polyoxyethylenes as hydrophilic groups are $C_{4-14}$ fatty acid glycerides having polyoxyethylene chains in which the number of repetitions of ethylene oxide units is 2 to 20 as hydrophilic groups, and the like, such as a combination of polyoxyethylene-hydrogenated castor oil and polyethylene glycol-caprylic acid/capric acid glycerides, a combination of polyoxyethylene(40)-hydrogenated castor oil and polyethylene glycol(8)-caprylic acid/capric acid glycerides, and the like.

In the semisolid or liquid oral pharmaceutical composition comprising two or more surfactants having different molecular weights, the content of the surfactants is 10 wt% or more with respect to the entire pharmaceutical composition, preferably 20 wt% or more.

In addition, when the two or more surfactants having different molecular weights are long-chain fatty acid glycerides having long-chain polyoxyethylenes as hydrophilic groups, and medium-chain fatty acid glycerides having short-chain polyoxyethylenes as hydrophilic groups, the respective blending ratios in the pharmaceutical composition are 1:10 to 10:1, preferably 1:5 to 5:1.

Although there are no particular restrictions on medicinal components that may be contained in the pharmaceutical composition of the present invention, the present invention has, particularly for a hardly water-soluble or water-insoluble active ingredient, superior effects in terms of increasing absorbability in the digestive tract and improving bioavailability of active ingredient when administered orally.

The term "hardly water-soluble or water-insoluble" in the "hardly water-soluble or water-insoluble active ingredient" mentioned above denotes a solubility of less than 10 mg/mL in water at 25°C, preferably less than 0.1 mg/mL. The solubility may be measured by a common method.

Examples of the "5- to 6-membered ring" in the "optionally substituted 5- to 6-membered ring" represented by $R^1$ in formula (I) above include groups formed by removing one hydrogen atom from 6-membered aromatic hydrocarbons such as benzene; 5- to 6-membered aliphatic hydrocarbons such as cyclopentane, cyclohexane, cyclopentene, cyclohexene, cyclopentadiene, cyclohexadiene; 5- to 6-membered aromatic heterocyclic ring having 1 to 4 of 1 to 2 kinds of hetero atoms selected from nitrogen atom, sulfur atom and oxygen atom such as furan, thiophene, pyrrole, imidazoles, pyrazole, thiazole, oxazole, isothiazole, isoxazole, tetrazole, pyridine, pyrazine, pyrimidine, pyridazine, triazole; and 5- to 6-membered non-aromatic heterocyclic ring having 1 to 4 of 1 to 2 kinds of hetero atoms selected from nitrogen atom, sulfur atom and oxygen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, and the like. However, among these groups, benzene, furan, thiophene, pyridine, cyclopentane, cyclohexane, pyrrolidine, piperidine, piperazine, morpholine, thiomorpholine, tetrahydropyran (preferably 6-membered ring), and the like are preferred for the "5- to 6-membered ring", inter alia, benzene is preferred.

Examples of the "substituents" optionally possessed by the "5- to 6-membered rings" of the "optionally substituted 5- to 6-membered rings" represented by $R^1$ include a halogen atom, nitro, cyano, optionally substituted alkyl, optionally substituted cycloalkyl, optionally substituted hydroxyl group, optionally substituted thiol group (where the sulfur atom may be oxidized to form an optionally substituted sulfinyl group or optionally substituted sulfonyl), optionally substituted amino group, optionally substituted acyl group, optionally esterified carboxyl group, and optionally substituted aromatic group.

Examples of the halogen as substituents in $R^1$ include fluorine, chlorine, bromine, and iodine, inter alia a fluorine and chlorine is preferred.

Examples of the alkyl in the optionally substituted alkyl as a substituent in $R^1$ include linear or branched $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower ($C_1$-$_6$) alkyl. Examples of the substituent in the optionally substituted alkyl include a halogen (e.g., fluorine, chlorine, bromine, iodine, or then like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkox-

ycarbonyl, carbamoyl, mono-$C_{1-4}$ alkylcarbamoyl, di-$C_{1-4}$ alkylcarbamoyl, or the like), $C_{1-4}$ alkoxy that may be halogenated (e.g., methoxy, ethoxy, propoxy, butoxy, trifluoromethoxy, trifluoroethoxy or the like), $C_{1-4}$ alkoxy-$C_{1-4}$ alkoxy that may be halogenated (e.g., methoxymethoxy, methoxyethoxy, ethoxyethoxy, trifluoromethoxyethoxy, trifluoroethoxyethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.) or the like, where the number of substituents is preferably 1 to 3.

Examples of the cycloalkyl for the optionally substituted cycloalkyl as a substituent in $R^1$ include $C_{3-7}$ cycloalkyl such as cyclopropyl, cyclobutyl, cyclopentyl, cyclohexyl, cycloheptyl, and the like. Examples of the substituent in the optionally substituted cycloalkyl include a halogen, (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$-alkylamino, 5- to 6-membered cyclic amino such as tetrahydropyrrole, piperazine, piperidine, morpholine, thiomorpholine, pyrrole, imidazole, and or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, and the like), $C_{1-4}$ alkoxy that may be halogenated (e.g., methoxy, ethoxy, propoxy, butoxy, trifluoromethoxy, trifluoroethoxy, or the like), $C_{1-4}$ alkoxy-$C_{1-4}$ alkoxy that may be halogenated (e.g., methoxymethoxy, methoxyethoxy, ethoxyethoxy, trifluoromethoxyethoxy, trifluoroethoxyethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, and the number of substituents is preferably 1 to 3.

Examples of the substituent in the optionally substituted hydroxyl group as a substituent in $R^1$ include:

(1) an optionally substituted alkyl (e.g., $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower ($C_{1-6}$) alkyl);
(2) an optionally substituted cycloalkyl that may comprise heteroatoms (e.g., $C_{3-7}$ cycloalkyl such as cyclopropyl, cyclobutyl, cyclopentyl, cyclohexyl and cycloheptyl; saturated 5- to 6-membered heterocyclic group having 1 to 2 heteroatoms such as tetrahydrofuranyl, tetrahydrothienyl, pyrrolidinyl, pyrazolidinyl, piperidyl, piperazinyl, morpholinyl, thiomorpholinyl, tetrahydropyranyl, tetrahydrothiopyranyl (preferably tetrahydropyranyl, etc.); or the like);
(3) an optionally substituted alkenyl (e.g., alkenyl having 2 to 10 carbons such as allyl, crotyl, 2-pentenyl, 3-hexenyl, preferably a lower ($C_2$-$_6$) alkenyl);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) an optionally substituted aralkyl (e.g., phenyl-$C_{1-4}$ alkyl (e.g., benzyl, phenethyl, etc.) or the like);
(6) a formyl or optionally substituted acyl (e.g., an alkanoyl having 2 to 4 carbons (e.g., acetyl, propionyl, butyryl, isobutyryl, or the like), alkylsulfonyl having 1 to 4 carbons (e.g., methanesulfonyl, ethanesulfonyl, etc.), or the like); or
(7) an optionally substituted aryl (e.g., phenyl, naphthyl, etc.), and the like; and

examples of the substituents that may be possessed by the (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, and (7) optionally substituted aryl mentioned above include a halogen, (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), $C_{1-4}$ alkyl that may be halogenated (e.g., trifluoromethyl, methyl, ethyl, or the like), optionally halogenated $C_{1-6}$ alkoxy (e.g., methoxy, ethoxy, propoxy, butoxy, trifluoromethoxy, trifluoromethoxy, or the like; preferably an optionally halogenated $C_{1-4}$ alkoxy), formyl, $C_{2-4}$ alkanoyl (e.g., acetyl, propionyl, or the like), $C_{1-4}$ alkylsulfonyl (e.g., methanesulfonyl, ethanesulfonyl, or the like), optionally substituted 5- to 6-membered aromatic heterocycle (e.g., 5- to 6-membered aromatic heterocyclic ring having 1 to 4 of 1 to 2 kinds of heteroatoms selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine and triazole; where examples of the substituent of the aforementioned heterocyclic ring include a halogen, (e.g., fluorine, chlorine, bromine, iodine, or the like), nitro, cyano, hydroxyl, thiol, amino, carboxyl, optionally halogenated $C_{1-4}$ alkyl (e.g., trifluoromethyl, methyl, ethyl, or the like), optionally halogenated $C_{1-4}$ alkoxy (e.g., methoxy, ethoxy, propoxy, butoxy, trifluoromethoxy, trifluoroethoxy, or the like), formyl, $C_{2-4}$ alkanoyl (e.g., acetyl, propionyl, or the like), $C_{1-4}$ alkylsulfonyl (e.g. a methanesulfonyl, ethanesulfonyl, etc.) or the like, where the number of substituents is preferably 1 to 3), or the like, and the number of the substituents is preferably 1 to 3.

The substituent for the optionally substituted thiol group as a substituent in $R^1$ is exemplified by those for the above "substituents in the optionally substituted hydroxyl group as a substituent in $R^1$", and among these, examples include:

(1) an optionally substituted alkyl (e.g., $C_{1-10}$ alkyl such as methyl, ethyl, propyl, isopropyl, butyl, isobutyl, sec-butyl,

tert-butyl, pentyl, isopentyl, neopentyl, hexyl, heptyl, octyl, nonyl and decyl, and preferably a lower ($C_{1-6}$) alkyl or the like);
(2) an optionally substituted cycloalkyl (e.g., $C_{3-7}$ cycloalkyl such as cyclopropyl, cyclobutyl, cyclopentyl, cyclohexyl, cycloheptyl, or the like);
(3) an optionally substituted aralkyl (e.g., phenyl-$C_{1-4}$ alkyl (e.g., benzyl or phenethyl) or the like); and
(4) an optionally substituted aryl (e.g., phenyl, naphthyl, etc.), and the like, and

examples of the substituent that may be possessed by the above-mentioned (1) optionally substituted alkyl, (2) optionally substituted cycloalkyl, (3) optionally substituted aralkyl and (4) optionally substituted aryl include a halogen, (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl or di-$C_{1-4}$ alkylcarbamoyl), $C_{1-4}$ alkoxy that may be halogenated (e.g., methoxy, ethoxy, propoxy, butoxy, trifluoromethoxy, trifluoroethoxy, or the like), $C_{1-4}$ alkoxy-$C_{1-4}$ alkoxy that may be halogenated (e.g., methoxymethoxy, methoxyethoxy, ethoxyethoxy, trifluoromethoxyethoxy, trifluoroethoxyethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, where the number of substituents is preferably 1 to 3.

Examples of the substituent for the optionally substituted amino group as a substituent in $R^1$ include amino groups having one to two of the same substituents as the "substituents in the optionally substituted hydroxyl group as a substituent in $R^1$" above, and among these, preferred examples include

(1) an optionally substituted alkyl (e.g., $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower ($C_{1-6}$) alkyl, or the like);
(2) an optionally substituted cycloalkyl (e.g., $C_{3-7}$ cycloalkyl such as cyclopropyl, cyclobutyl, cyclopentyl, cyclohexyl, cycloheptyl, or the like);
(3) an optionally substituted alkenyl (e.g., alkenyl having 2 to 10 carbons such as allyl, crotyl, 2-pentenyl and 3-hexenyl, preferably a lower ($C_{2-6}$) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl and 2-cyclohexenylmethyl) or the like;
(5) a formyl or optionally substituted acyl (e.g., an alkanoyl having 2 to 4 carbons (e.g., acetyl, propionyl, butyryl, or isobutyryl) and alkylsulfonyl having 1 to 4 carbons (e.g., methanesulfonyl or ethanesulfonyl) or the like; and
(6) an optionally substituted aryl (e.g., phenyl, naphthyl, etc.), and the like; and

examples of the substituents that may be possessed by the above-mentioned (1) optionally substituted alkyl, (2) optionally substituted cycloalkyl, (3) optionally substituted alkenyl, (4) optionally substituted cycloalkenyl, (5) optionally substituted acyl, and (6) optionally substituted aryl include a halogen, (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), optionally halogenated $C_{1-4}$ alkoxy (e.g., methoxy, ethoxy, propoxy, butoxy, trifluoromethoxy, trifluoroethoxy, or the like), optionally halogenated $C_{1-4}$ alkoxy-$C_{1-4}$ alkoxy (e.g., methoxymethoxy, methoxyethoxy, ethoxyethoxy, trifluoromethoxyethoxy, trifluoroethoxyethoxy, or the like), formyl, $C_{2-4}$ alkanoyl (e.g., acetyl, propionyl, or the like) or $C_{1-4}$ alkylsulfonyl (e.g., methanesulfonyl, ethanesulfonyl, etc.), and the like, where the number of substituents is preferably 1 to 3.

In addition, with the optionally substituted amino groups as substituents in $R^1$, two of the substituents on the amino group may be bonded together to form a cyclic amino group (e.g., cyclic amino group having a bond on the nitrogen atom, formed by removing one hydrogen atom from the ring constituent nitrogen atom of a 5- to 6-membered ring such as tetrahydropyrrole, piperazine, piperidine, morpholine, thiomorpholine, pyrrole, imidazole, or the like). This cyclic amino group may have substituents, and examples of the substituents include a halogen, (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), optionally halogenated $C_{1-4}$ alkoxy (e.g., methoxy, ethoxy, propoxy, butoxy, trifluoromethoxy, trifluoroethoxy, or the like), optionally halogenated $C_{1-4}$ alkoxy-$C_{1-4}$ alkoxy (e.g., methoxymethoxy,

methoxyethoxy, ethoxyethoxy, trifluoromethoxyethoxy, trifluoroethoxyethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, where the number of substituents is preferably 1 to 3.

Examples of the optionally substituted acyl as a substituent in $R^1$ include those in which a carbonyl group or sulfonyl group is bonded with:

(1) a hydrogen;
(2) an optionally substituted alkyl (e.g., $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower ($C_{1-6}$) alkyl or the like);
(3) an optionally substituted cycloalkyl (e.g., $C_{3-7}$ cycloalkyl such as cyclopropyl, cyclobutyl, cyclopentyl, cyclohexyl, cycloheptyl, or the like);
(4) an optionally substituted alkenyl (e.g., alkenyl having 2 to 10 carbons such as allyl, crotyl, 2-pentenyl, 3-hexenyl, and preferably a lower ($C_{2-6}$) alkenyl or the like);
(5) an optionally substituted cycloalkenyl (e.g., cycloalkenyl having 3 to 7 carbons such as 2-cyclopentenyl, 2-cyclohexyl, 2-cyclopentenylmethyl, 2-cyclohexenylmethyl, or the like);
(6) those wherein an optionally substituted 5- to 6-membered monocyclic aromatic group (e.g., phenyl, pyridyl, etc.), and the like is linked with carbonyl group or sulfonyl group (e.g., formyl, acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, or the like). Examples of the substituent that may be possessed by the above-mentioned (2) optionally substituted alkyl, (3) optionally substituted cycloalkyl, (4) optionally substituted alkenyl, (5) optionally substituted cycloalkenyl, or (6) optionally substituted 5-to 6-membered monocyclic aromatic group include a halogen, (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), optionally halogenated $C_{1-4}$ alkoxy (e.g., methoxy, ethoxy, propoxy, butoxy, trifluoromethoxy, trifluoroethoxy, or the like), optionally halogenated $C_{1-4}$ alkoxy-$C_{1-4}$ alkoxy (e.g., methoxymethoxy, methoxyethoxy, ethoxyethoxy, trifluoromethoxyethoxy, trifluoroethoxyethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, where the number of substituents is preferably 1 to 3.

Examples of the optionally esterified carboxyl group as the substituents in $R^1$ include those wherein a carbonyloxy group is bonded with

(1) a hydrogen;
(2) an optionally substituted alkyl (e.g. $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and preferably a lower ($C_{1-6}$) alkyl or the like);
(3) an optionally substituted cycloalkyl (e.g., $C_{3-7}$ cycloalkyl such as cyclopropyl, cyclobutyl, cyclopentyl, cyclohexyl, cycloheptyl, or the like);
(4) an optionally substituted alkenyl (e.g., alkenyl having 2 to 10 carbons such as allyl, crotyl, 2-pentenyl, 3-hexenyl, and preferably a lower ($C_{2-6}$) alkenyl or the like);
(5) an optionally substituted cycloalkenyl (e.g., cycloalkenyl having 3 to 7 carbons such as 2-cyclopentenyl, 2-cyclohexyl, 2-cyclopentenylethyl, 2-cyclohexenylmethyl, or the like); or
(6) an optionally substituted aryl (e.g., phenyl, naphthyl, etc.), and the like, and preferably a carboxyl, lower ($C_{1-6}$) alkoxycarbonyl, aryloxycarbonyl (e.g., methoxycarbonyl, ethoxycarbonyl, propoxycarbonyl, phenoxycarbonyl, naphthoxycarbonyl, etc.), and the like. Examples of the substituent that the above (2) optionally substituted alkyl, (3) optionally substituted cycloalkyl, (4) optionally substituted alkenyl, (5) optionally substituted cycloalkenyl and (6) optionally substituted aryl may have, include a halogen, (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), optionally halogenated $C_{1-4}$ alkoxy (e.g., methoxy, ethoxy, propoxy, butoxy, trifluoromethoxy, trifluoroethoxy, or the like), optionally halogenated $C_{1-4}$ alkoxy-$C_{1-4}$ alkoxy (e.g., methoxymethoxy, methoxyethoxy, ethoxyethoxy, trifluoromethoxyethoxy, trifluoroethoxyethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, where the number of substituents is preferably 1 to 3.

Examples of the aromatic group in the optionally substituted aromatic groups as substituents in $R^1$ include 5- to 6-membered homocyclic or heterocyclic aromatic group such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl and triazolyl; condensed heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine; and the like. Examples of the substituent for these aromatic groups include a halogen (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), optionally halogenated $C_{1-4}$ alkyl (e.g., trifluoromethyl, methyl, ethyl, or the like), optionally halogenated $C_{1-4}$ alkoxy (e.g., methoxy, ethoxy, propoxy, butoxy, trifluoromethoxy, trifluoroethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, where the number of substituents is preferably 1 to 3.

These substituents in $R^1$ may be substituted with the same or different 1 to 4 (preferably 1 to 2) at any of positions on the ring. In addition, when the "5- to 6-membered ring" of the "optionally substituted 5- to 6-membered ring" represented by $R^1$ has two or more substituents, two of these substituents may be bonded together to form a group such as a lower ($C_{1-6}$) alkylene (e:g., trimethylene, tetramethylene, or the like), lower ($C_{1-6}$) alkyleneoxy (e.g., $-CH_2-O-CH_2$ $-O-CH_2-CH_2-$, $-O-CH_2-CH_2-CH_2-$, $-O-CH_2-CH_2-CH_2-CH_2-$, $-O-C(CH_3)$ $(CH_3)-CH_2-CH_2-$, or the like), lower ($C_{1-6}$) alkylenethio (e.g., $-CH_2-S-CH_2-$, $-S-CH_2-CH_2-$, $-S-CH_2-CH_2-CH_2-$, $-S-CH_2-CH_2-CH_2-CH_2-$, $-S-C(CH_3)$ $(CH_3)-CH_2-CH_2-$, or the like), lower ($C_{1-6}$) alkylenedioxy (e.g., $-$ $O-CH_2-O-$, $-O-CH_2-CH_2-O-$, $-O-CH_2-CH_2-CH_2-O-$, or the like), lower ($C_{1-6}$) alkylenedithio (e.g., $-S-CH_2-S-$, $-S-CH_2-CH_2-S-$, $-S-CH_2-CH_2-CH_2-S-$, or the like), oxy-lower ($C_{1-6}$) alkyleneamino (e.g., $-O-CH_2-NH-$, $-O-CH_2-CH_2-NH-$, or the like), oxy-lower ($C_{1-6}$) alkylenethio (e.g., $-O-CH_2-S-$, $-O-CH_2-CH_2-S-$, or the like), lower ($C_{1-6}$) alkyleneamino (e.g., $-NH-CH_2-CH_2-$, $-NH-CH_2-CH_2-CH_2-$, or the like), lower ($C_{1-6}$) alkylenediamino (e.g., $-NH-CH_2-NH-$, $-NH-CH_2-CH_2-NH-$, or the like), thia-lower ($C_{1-6}$) alkyleneamino (e.g., $-S-CH_2-NH-$, $-$ $S-CH_2-CH_2-NH-$, or the like), lower ($C_{2-6}$) alkenylene (e.g., $-CH_2-CH=CH-$, $-CH_2-CH_2-CH=CH-$, $-CH_2-CH=CH-CH_2$, or the like), lower ($C_{4-6}$) alkadienylene (e.g., $-CH=CH-CH=CH-$, etc.), and the like.

In addition, the divalent groups that are formed by combining two substituents of $R^1$ may have 1 to 3 substituents similar to the "substituents" that may be possessed by the "5- to 6-membered ring" of the "optionally substituted 5- to 6-membered ring" (e.g., halogen atom, nitro, cyano, optionally substituted alkyl, optionally substituted cycloalkyl, optionally substituted hydroxyl, optionally substituted thiol group (where the sulfur atom may be oxidized, and may form an optionally substituted sulfinyl group or optionally substituted sulfonyl group), optionally substituted amino group, optionally substituted acyl, optionally esterified or amidated carboxyl group, optionally substituted aromatic group or the like).

Specific examples of the "substituents" that the "5-to 6-membered ring" of the "optionally substituted 5- to 6-membered ring" represented by $R^1$ may have, include a ($C_{1-4}$) alkyl that may be halogenated or may be alkoxylated with a ($C_{1-4}$) alkoxy (e.g., methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, or the like); a lower ($C_{1-4}$) alkoxy that may be halogenated or may be alkoxylated with a ($C_{1-4}$) alkoxy (e.g., methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, or the like); halogen (e.g., fluorine, chlorine, or the like); nitro; cyano; amino that may be substituted with 1 to 2 lower ($C_{1-4}$) alkyl groups, formyl groups, or lower ($C_{2-4}$) alkanoyl groups (e.g. an amino, methylamino, dimethylamino, formylamino, acetylamino, or the like); 5- to 6-membered cyclic amino group (e.g., 1-pyrrolidinyl, 1-piperazinyl, 1-piperidinyl, 4-morpholino, 4-thiomorpholino, 1-imidazolyl, 4-tetrahydropyranyl, etc.); and the like.

Examples of the "divalent group wherein the number of atoms constituting the straight chain moiety is 1 to 4" denoted by $X^1$ and $X^2$ include $-(CH_2)_{a'}-$ (where a' denotes an integer of 1 to 4 (with an integer of 1 to 2 being preferred)), $-(CH_2)_{b'}-X^3-$ (where b' denotes integer of 0 to 3 (preferably 0 or 1), and $X^3$ denotes an optionally substituted imino group (e.g., an imino group that may be substituted with a lower ($C_{1-6}$) alkyl, lower ($C_{3-7}$) cycloalkyl, formyl, lower ($C_{2-7}$) alkanoyl, lower ($C_{1-6}$) alkoxycarbonyl, or the like), carbonyl group, oxygen atom, or optionally oxidized sulfur atom(e.g., $-S(O)_m-$ (where m denotes an integer of 0 to 2)), $-CH=CH-$, $-C\equiv C-$, $-CO-NH-$, $-$ $SO_2-NH-$, and the like. The bonding of these groups to ring A or ring B can be achieved by either the left or right bond, but with $X^1$, it is preferable for bonding with ring A to occur via the right-side bond, and with $X^2$, it is preferable for bonding with ring B to occur via the left-side bond.

It is preferable for $X^1$ to be a bond, $-(CH_2)_{b'}-O-$(where b' denotes an integer of 0, 1, or 2 (preferably 0 or 1)), $-C\equiv C-$, or the like, with a bond being more preferred.

$X^2$ is preferably $-(CH_2)_{a'}-$ (where a denotes an integer of 1 to 2), $-(CH_2)_{b'}-X^3-$ (where b' denotes an integer of 0 or 1 and $X^3$ denotes an optionally substituted imino group, carbonyl group, oxygen atom, or optionally oxidized sulfur

atom), -CH=CH, -CO-NH-, $-SO_2-NH-$, or the like, with -CO-NH-being more preferred.

The divalent group represented by $X^1$ and $X^2$ may have a substituent at any position (preferably on a carbon atom), and examples of the substituents are any substituent that can be bonded to the divalent chain that constitutes the straight chain moiety. For example, a lower ($C_{1-6}$) alkyl (e.g., methyl, ethyl, propyl, isopropyl, butyl, isobutyl, sec-butyl, tert-butyl, pentyl, isopentyl, neopentyl, hexyl, or the like), lower ($C_{3-7}$) cycloalkyl (e.g., cyclopropyl, cyclobutyl, cyclopentyl, cyclohexyl, cycloheptyl, or the like), formyl, lower ($C_{2-7}$) alkanoyl (e.g., acetyl, propionyl, butyryl, or the like), optionally esterified phosphono group, optionally esterified carboxyl group, hydroxyl group, oxo, and the like, and preferably a lower alkyl having 1 to 6 carbons (preferably a $C_{1-3}$ alkyl), hydroxyl, oxo, and the like.

Examples of the optionally esterified phosphono groups include -P(O) ($OR^7$) ($OR^8$) (wherein, $R^7$ or $R^8$ each denote a hydrogen, alkyl group having 1 to 6 carbons, or cycloalkyl group having 3 to 7 carbons, and $R^7$ and $R^8$ may be bonded together to form a 5- to 7-membered ring).

In the above formula, examples of the alkyl groups having 1 to 6 carbons represented by $R^7$ and $R^8$ include methyl, ethyl, propyl, isopropyl, butyl, isobutyl, sec-butyl, tert-butyl, pentyl, isopentyl, neopentyl, hexyl, and the like, and examples of cycloalkyls having 3 to 7 carbons include a cyclopropyl, cyclobutyl, cyclopentyl, cyclohexyl, cycloheptyl, and the like, and chain lower alkyls having 1 to 6 carbons are preferred, and lower alkyls having 1 to 3 carbons are more preferred. $R^7$ and $R^8$ may be the same or different, but it is preferable for them to be the same. When $R^7$ and $R^8$ are bonded together to form a 5- to 7-membered ring, $R^7$ and $R^8$ are bonded together to form a linear $C_{2-4}$ alkylene side chain represented by $-(CH_2)_2-$, - $(CH_2)_3-$, or $-(CH_2)_4-$. This side chain may have substituents, and examples of the substituents include a hydroxyl group, halogen, and the like.

Examples of the esterified carboxyl groups for the optionally esterified carboxyl group include a group produced by bonding a carboxyl group with an alkyl group having 1 to 6 carbons or a cycloalkyl group having 3 to 7 carbons , examples of which include methoxycarbonyl, ethoxycarbonyl propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, and the like.

Examples of the "5- to 6-membered rings" of the "optionally substituted 5- to 6-membered ring" represented by A in formula (I) above include a 5- to 6-membered saturated or unsaturated alicyclic hydrocarbons such as $C_{5-6}$ cycloalkane (e.g., cyclopentane, cyclohexane, or the like), $C_{5-6}$ cycloalkene (e.g., 1-cyclopentene, 2-cyclopentene, 3-cyclopentene, 2-cyclohexene, 3-cyclohexene, or the like), $C_{5-6}$ cycloalkadiene (e.g., 2,4-cyclopentadiene, 2,4-cyclohexadiene, 2,5-cyclohexadiene, or the like) ; 6-membered aromatic hydrocarbons such as benzene; 5- to 6-membered aromatic heterocyclic rings, or saturated or unsaturated non-aromatic heterocyclic ring (aliphatic heterocyclic ring), each of which contains at least 1 (preferably 1 to 4, and more preferably 1 or 2) of 1 to 3 kinds (preferably 1 or 2 kinds) of heteroatom selected from an oxygen atom, sulfur atom, nitrogen atom, and the like; and the like.

Herein, examples of the "aromatic heterocyclic rings" include a 5- to 6-membered aromatic monocyclic heterocyclic ring (e.g., furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, or the like), and examples of the "non-aromatic heterocyclic rings" include 5- to 6-membered saturated or unsaturated non-aromatic heterocyclic rings (aliphatic heterocyclic ring) such as pyrrolidine, tetrahydrofuran, thiolane, piperidine, tetrahydropyran, morpholine, thibmorpholine, piperazine, pyran, oxepine, thiepine, azepine or the like, or a 5- to 6-membered non-aromatic heterocyclic ring wherein part or all of the double bonds of the above-mentioned aromatic monocyclic heterocyclic ring are saturated or the like.

Examples of the "5- to 6-membered rings" of the "optionally substituted 5- to 6-membered ring" represented by A are preferably 5- to 6-membered aromatic rings, and more preferably benzene, furan, thiophene, pyrrole, pyridine (preferably 6-membered rings), and the like, with benzene being the most preferred.

Examples of the "substituents" that the "5- to 6-membered ring" of the "optionally substituted 5- to 6-membered ring" represented by A may have, include substituents similar to the "substituents" that the "5- to 6-membered ring" of the "optionally substituted 5- to 6-membered ring" may have. In addition, the substituents for A may be substituted with the same or different 1 to 4 (preferably 1 to 2) at any of positions on the ring, and substituents may be present at any position if the position is a substitutable position, regardless of whether it is a position represented by $E_1$ and $E_2$ or another position.

Examples of the lower alkyl groups of the "optionally substituted lower alkyl group" represented by $R^3$ above include $C_{1-6}$ alkyl such as methyl, ethyl, propyl, isopropyl, butyl, isobutyl, sec-butyl, tert-butyl, pentyl, isopentyl, neopentyl, hexyl, and the like.

Examples of the lower alkoxy groups of the "optionally substituted lower alkoxy group" represented by $R^3$ above include $C_{1-6}$ alkoxy such as methoxy, ethoxy, propoxy, butoxy.

Examples of the substituents that the "optionally substituted lower alkyl group" and "optionally substituted lower alkoxy group" may have, include a halogen (e.g., fluorine, chlorine, bromine, iodine), hydroxyl group, amino group, mono (lower alkyl)amino, di(lower alkyl)amino, lower alkanoyl, and the like.

Examples of the lower alkyl in the mono(lower alkyl)amino and di(lower alkyl)amino include the same groups as the lower alkyl group of the "optionally substituted lower alkyl group" represented by $R^3$ above.

Examples of the lower alkanoyl are $C_{2-6}$ alkanoyl such as acetyl, propionyl, butyryl, isobutyryl.

Examples of the "halogen atom" represented by $R^3$ above include fluorine, chlorine, bromine, iodine, and the like.

Among these groups, an optionally substituted lower $C_{1-6}$ alkyl group or halogen atom is preferred for $R^3$, and an optionally substituted methyl group or halogen atom is particularly preferred.

Examples of the "8- to 10-membered ring" of the "optionally substituted 8- to 10-membered ring" represented by B in formula (I) above include 8- to 10-membered rings having substituents at any substitutable position represented by the formula:

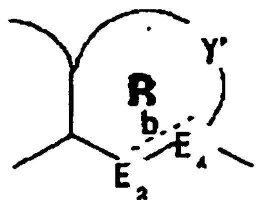

wherein, Y' denotes a divalent group, and the other symbols have the same designations as above.

In the above formula, the divalent group represented by Y' denotes a divalent group whereby ring B forms an optionally substituted 8- to 10-membered ring, and examples include:

(1) $-Alk_{a1}-O-Alk_{a2}-$ (where $Alk_{a1}$ and $Alk_{a2}$ each denote a bond or a divalent linear hydrocarbon group having 1 to 5 carbons, provided that the sum of the carbon numbers of $Alk_{a1}$ and $Alk_{a2}$ is 5 or less),

(2) $-Alk_{b1}-S(O)_m-Alk_{b2}-$ (where m denotes an integer of 0, 1, or 2; $Alk_{b1}$ and $Alk_{b2}$ each denote a bond or a divalent linear hydrocarbon group having 1 to 5 carbons; provided that the sum of the carbon numbers of $Alk_{b1}$ and $Alk_{b2}$ is 5 or less),

(3) $-Alk_{d1}-$ (where $Alk_{d1}$ denotes a divalent linear hydrocarbon group having 4 to 6 carbons),

(4) $-Alk_{e1}-NH-Alk_{e2}-$ ($Alk_{e1}$ and $Alk_{e2}$ each denote a bond or a divalent linear hydrocarbon group having 1 to 5 carbons, provided that the sum of the carbon numbers of $Alk_{e1}$ and $Alk_{e2}$ is 5 or less), $-Alk_{e6}-N=CH-Alk_{e7}-$, $-Alk_{e7}-CH=N-Alk_{e6}-$, $-Alk_{e6}-N=N-Alk_{e7}-$ (where $Alk_{e6}$ and $Alk_{e7}$ each denote a bond or a divalent linear hydrocarbon group having 1 to 4 carbons, provided that the sum of the carbon numbers of $Alk_{e6}$ and $Alk_{e7}$ is 4 or less), and the like.

Examples of these divalent linear hydrocarbon groups include divalent groups such as $-CH_2-$, $-(CH_2)_2-$, $-(CH_2)_3-$, $-(CH_2)_4-$, $-(CH_2)_5-$, $-(CH_2)_6$, $-CH=$, $-CH=CH-$, $-CH=CH-CH_2-$, $-CH_2CH=CH-$, $-CH=CH-CH=CH-$, $=CH-CH=CH-$, $-CH_2-CH=CH-CH_2-$, $-CH=CH-(CH_2)_2-$, $-CH=CH-(CH_2)_3-$, $-CH=CH-(CH_2)_4-$, and the like.

Specific examples of Y' include $-O-(CH_2)_3-$, $-O-(CH_2)_4-$, $-O-(CH_2)_5-$, $-CH_2-O-(CH_2)_2-$, $-O-CH=CH-CH_2-$, $S(O)_m-(CH_2)_3-$(where m denotes an integer of 0 to 2), $-S(O)_m-(CH_2)_4-$(where m denotes an integer of 0 to 2), $-S(O)_m-(CH_2)_5-$(where m denotes an integer of 0 to 2), $-CH_2-S(O)_m-(CH_2)_2-$(where m denotes an integer of 0 to 2), $-S(O)_m-CH=CH-CH_2-$(where m denotes an integer of 0 to 2), $-(CH_2)_4-$, $-(CH_2)_5-$, $-(CH_2)_6-$, $-CH=CH-CH=CH-$, $-CH=CH-(CH_2)_2-$, $-NH-(CH_2)_3-$, $-NH-(CH_2)_4-$, $-NH-(CH_2)_5-$, $-CH_2-NH-(CH_2)_2-$, $-NH-CH=CH-CH_2-$, $-N=CH-Ch=CH-$, $-CH=N-(CH_2)_2-$, $-CH=N-CH=CH-$, $-N=N-(CH_2)_2-$, $-N=N-CH=CH-$, $-CH=N-N=CH-$ (each denoting a bond that starts on ring A), and the like. An 8-membered ring is preferable for ring B.

In addition, the divalent group may have substituents, and examples of the substituents include an oxo group and the same substituents as the "substituents" that the "5- to 6-membered ring" of the "optionally substituted 5- to 6-membered ring" represented by $R^1$ may have, and among these, a lower ($C_{1-3}$) alkyl (e.g., methyl, ethyl, propyl, or the like), phenyl, oxo, hydroxyl group, and the like are preferred. The substituents of the divalent group may be the same or different, and 1 to 6 (preferably 1 to 2) of them may be substituted. Any substitution position is acceptable, provided that bonding to the divalent group is possible.

Examples of the "substituents" that the "8- to 10-membered ring" of the "optionally substituted 8- to 10-membered ring" represented by B may have, are an oxo group and the same substituents as the "substituents" that the "5- to 6-membered ring" of the "optionally substituted 5-to 6-membered ring" represented by $R^1$ may have.

Examples of the divalent group represented by Y are preferably $-O-(CH_2)_3-$, $-O-(CH_2)_4-$ $-O-(CH_2)_5-$, $-S(O)_m-(CH_2)_3-$(m denotes an integer of 0 to 2), $-S(O)_m-(CH_2)_4-$ (m denotes an integer of 0 to 2), $-S(O)_m-(CH_2)_5-$ (m denotes an integer of 0 to 2), $-(CH_2)_4-$, $-(CH_2)_5$, $-(CH_2)_6-$, and a group having a divalent group represented by the formula $-N(R^o)-$ (wherein, $R^o$ denotes a hydrogen atom or a substituent) such as $-NH-(CH_2)_3-$, $-NH-(CH_2)_4-$ and $-NH-(CH_2)_5-$ in the main chain. Inter alia, the group having a divalent group represented by the formula $-N(R^o)-$ (wherein, $R^o$ denotes a hydrogen atom or a substituent) in the main chain is preferred.

Preferred examples of $R^o$ include a hydrogen atom, optionally substituted hydrocarbon group, heterocyclic group that may be substituted, optionally substituted hydroxyl group, optionally substituted thiol group (where the sulfur

atom may be oxidized to form an optionally substituted sulfinyl group or optionally substituted sulfonyl group), optionally substituted amino group, optionally esterified or amidated carboxyl group, optionally substituted acyl group, and the like, and more preferably a hydrogen atom, optionally substituted hydrocarbon group, optionally substituted heterocyclic group, optionally substituted acyl group, and the like.

Preferred modes for $R^o$ include a hydrogen atom, optionally substituted hydrocarbon group, and optionally substituted acyl group, and as the optionally substituted hydrocarbon group, preferred are an optionally halogenated or hydroxylated $C_{1-6}$ alkyl and an optionally halogenated or hydroxylated $C_{2-6}$ alkenyl. Preferred examples of the optionally substituted acyl groups include an optionally halogenated or hydroxylated $C_{1-4}$ alkylsulfonyl, formyl, optionally halogenated or hydroxylated $C_{2-5}$ alkanoyl, and the like, and $R^o$ is more preferably an optionally halogenated or hydroxylated $C_{1-4}$ alkyl, a formyl, an optionally halogenated or hydroxylated $C_{2-5}$ alkanoyl, and the like, inter alia, propyl, isobutyl, isobutenyl, or 3-hydroxy-2-methylpropyl is preferred. Another preferred mode for $R^o$ includes groups represented by the formula - $(CH_2)_s$-$R^x$ {wherein, s denotes 0 or 1, and $R^x$ denotes an optionally substituted 5- to 6-membered monocyclic aromatic group (e.g., the same groups as the "5- to 6-membered monocyclic aromatic groups" exemplified in the paragraph concerning ring A; preferably a phenyl, pyridyl, pyrazolyl, thiazolyl, oxazolyl, tetrazolyl, and the like, each of which may be substituted with a halogen, an optionally halogenated or hydroxylated $C_{1-4}$ alkyl or an optionally halogenated or hydroxylated $C_{1-4}$ alkoxy or the like)).

Examples of the "hydrocarbon group" of the "optionally substituted hydrocarbon group" include:

(1) an alkyl (e.g., $C_{1-10}$ alkyl such as methyl, ethyl, propyl, isopropyl, butyl, isobutyl, sec-butyl, tert-butyl, pentyl, isopentyl, neopentyl, hexyl, sec-butyl, tert-butyl, pentyl, isopentyl, neopentyl, hexyl, heptyl, octyl, nonyl, decyl, preferably a lower ($C_{1-6}$) alkyl, and more preferably a lower ($C_{1-4}$) alkyl or the like);
(2) a cycloalkyl (e.g., $C_{3-7}$ cycloalkyl such as cyclopropyl, cyclobutyl, cyclopentyl, cyclohexyl, cycloheptyl, or the like);
(3) an alkenyl (e.g., alkenyl having 2 to 10 carbons such as allyl, crotyl, 2-pentenyl, 3-hexenyl, and preferably a lower ($C_{2-6}$) alkenyl or the like);
(4) a cycloalkenyl (e.g., cycloalkenyl having 3 to 7 carbons such as 2-cyclopentenyl, 2-cyclohexenyl, 2-cyclopentenylmethyl, 2-cyclohexenylmethyl);
(5) an alkynyl (e.g., alkynyl having 2 to 10 carbons such as ethynyl, 1-propynyl, 2-propynyl, 1-butynyl, 2-pentynyl, 3-hexynyl, preferably a ($C_{2-6}$) alkynyl or the like);
(6) an aralkyl (e.g., phenyl-$C_{1-4}$ alkyl (e.g., benzyl or phenethyl) or the like);
(7) an aryl (e.g., phenyl, naphthyl, or the like);
(8) a cycloalkyl-alkyl (e.g., $C_{3-7}$ cycloalkyl-$C_{1-4}$ alkyl such as cyclopropylmethyl, cyclobutylmethyl, cyclopentylmethyl, cyclohexylmethyl, cycloheptylmethyl, etc.); and the like; and the substituents that the above (1) alkyl, (2) cycloalkyl, (3)alkenyl, (4) cycloalkenyl, (5) alkynyl, (6) aralkyl, (7) aryl, and (8) cycloalkyl-alkyl may have, include a halogen (e.g., fluorine, chlorine, bromine, iodine, or the like) nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), $C_{1-4}$ alkyl that may be halogenated (e.g., trifluoromethyl, methyl, ethyl, or the like), $C_{1-4}$ alkoxy that may be halogenated (e.g., methoxy, ethoxy, propoxy, butoxy, trifluoromethoxy, trifluoroethoxy, or the like), $C_{1-4}$ alkylenedioxy (e.g., $-O-CH_2-O-$, $-O-CH_2-CH_2-O-$, or the like), sulfonamide that may be substituted (e.g., a group formed by bonding an optionally substituted amino group (e.g., amino, mono $C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like) with $-SO_2-$, or the like, ), formyl, $C_{2-4}$ alkanoyl (e.g., acetyl, propionyl, or the like), $C_{1-4}$ alkylsulfonyl (e.g., methanesulfonyl, ethanesulfonyl, or the like), optionally substituted heterocyclic group, and the like, where the number of substituents is preferably 1 to 3.

Examples of the "heterocyclic groups" pertaining to the optionally substituted heterocyclic group represented by $R^o$ and the "optionally substituted heterocyclic group" above include groups formed by the removal of one hydrogen atom from aromatic heterocycles or non-aromatic heterocycles. Examples of the aromatic heterocycles include a 5- to 6-membered aromatic heterocycle containing 1 to 4 of one or two kinds of heteroatoms selected from nitrogen atom, sulfur atom and oxygen atom such as furan, thiophene, pyrrole, imidazoles, pyrazole, thiazole, oxazole, isothiazole, isoxazole, tetrazole, pyridine, pyrazine, pyrimidine, pyridazine, triazole, oxadiazole, thiadiazole, and examples of the non-aromatic heterocycle include a 5- to 6-membered non-aromatic heterocycle having 1 to 4 of one or two kinds of heteroatoms selected from nitrogen atom, sulfur atom, and oxygen atom such as tetrahydrofuran, tetrahydrothiophene, dioxolane, dithiolane, oxathiolane, pyrrolidone, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, and non-aromatic heterocycle in which some or all of the bonds on the aromatic heterocycle are saturated bonds (preferably

aromatic heterocycles such as pyrazole, thiazole, oxazole, tetrazole), and the like.

Examples of the "optionally substituted hydroxyl group", "optionally substituted thiol group", "optionally substituted amino group", "optionally esterified carboxyl group," and "optionally substituted acyl group" represented by $R^o$ include the same groups as the "optionally substituted hydroxyl group", "optionally substituted thiol group", "optionally substituted amino group", "optionally esterified carboxyl group" and "optionally substituted acyl group" as the substituents that may be possessed by the "5-to 6-membered ring group" of the "optionally substituted 5-to 6-membered ring group" represented by $R^1$. Examples of the "optionally amidated carboxyl group" include groups wherein the "optionally substituted amino group" is linked with carbonyl group, preferably carbamoyl, mono-$C_{1-6}$ alkylcarbamoyl, di-$C_{1-6}$ alkylcarbamoyl, and the like.

The imino group represented by $Y^a$ that may have a formyl, optionally substituted $C_{1-6}$ alkyl, optionally substituted $C_{2-6}$ alkenyl, optionally substituted aryl, optionally substituted heterocyclic group, optionally substituted arylmethyl, or optionally substituted heterocyclic-methyl as substituents denotes groups within the definition of the groups described in relation to ($R^o$)-represented by Y. Among these groups, it is preferable for $R^o$ to be (1) a $C_{1-6}$ alkyl, (2) a $C_{2-6}$ alkenyl, (3) a $C_{6-10}$ aryl, (4) a $C_{6-10}$ aryl-methyl, (5) a heterocyclic group, or (6) a heterocyclic-methyl (wherein (1) and (2) may be substituted with halogen or hydroxyl group, and (3), (4), (5), and (6) may be substituted with a halogen, a $C_{1-6}$ alkyl optionally substituted with a halogen or hydroxyl group, or a $C_{1-6}$ alkoxy that may be substituted with a halogen or hydroxyl group).

In addition, the substituents of B may be the same or different, and 1 to 7 (preferably 1 to 2) may be substituted at any position (including $E_3$ and $E_4$), but it is preferable for the $E_3$ position to be unsubstituted.

In formula (I) above, compounds are preferred wherein $E_3$ and $E_4$ are each an optionally substituted carbon atom (preferably an unsubstituted carbon atom), and b is a double bond.

In formula (I) above, examples of the "divalent cyclic groups" represented by $Z^1$ are the same groups as the 5- to 6-membered ring of the "optionally substituted 5- to 6-membered ring" represented by $R^1$ or groups formed by the removal of two hydrogen atoms from a condensed aromatic heterocycle such as a benzofuran, indole, benzothiophene, benzoxazole, benzothiazole, indazole, benzimidazole, quinoline, isoquinoline, quinoxaline, phthalazine, quinazoline, cinnoline, imidazopyridine, and the like. Among these, divalent cyclic groups are preferred which are formed by the removal of two hydrogen atoms from benzene, furan, thiophene, pyridine, pyridazine, pyrimidine, benzimidazole, cyclopentane, cyclohexane, pyrrolidine, piperidine, piperazine, morpholine, thiomorpholine, tetrahydropyran, and the like; and divalent cyclic groups are particularly preferred which are formed by the removal of 2 hydrogen atoms from benzene, pyridine, pyridazine, benzimidazole, cyclohexane, or piperidine (preferably benzene).

The "divalent cyclic group" represented by $Z^1$ may have the same substituents as the "substituents" that the "5- to 6-membered ring" of the "optionally substituted 5- to 6-membered ring group" represented by $R^1$ may have. Among these, preferred substituents include a halogen atom (e.g., fluorine, chlorine, bromine, or the like), $C_{1-4}$ alkyl group that may be substituted with a halogen atom (e.g., methyl, ethyl, trifluoromethyl, trifluoroethyl, or the like), or $C_{1-4}$ alkoxy group that may be substituted with a halogen atom (e.g., methoxy, ethoxy, propoxy, trifluoromethoxy, trifluoroethoxy, or the like), but it is preferable not to have substituents except $X^2$ and $Z^2$. In addition, when $Z^1$ is a 6-membered divalent cyclic group (preferably phenylene), the substitution position on $Z^2$ is preferably the para-position of $X^2$. In addition, $Z^1$ is preferably a phenylene optionally having 1) a halogen atom, 2) a $C_{1-4}$ alkyl group that may be substituted with a halogen atom, or 3) a $C_{1-4}$ alkoxy group that may be substituted with a halogen atom, as a substituent, and a phenylene having a methyl group or trifluoromethyl group as a substituent is particularly preferred.

The divalent group represented by $Z^2$ in formula (I) above is represented, for example, by the formula $-Z^{2a}-W^1-Z^{2b}-$ ($Z^{2a}$ and $Z^{2b}$ each denote O, $S(O)_m$ (wherein m denotes 0, 1, or 2), an optionally substituted imino group ($-N(R^a)-$), or a bond, and $W^1$ denotes an optionally substituted alkylene group, optionally substituted alkenylene group, or a bond). When $Z^1$ is a benzene ring, for example, the bonding position of $Z^2$ may be any position but is preferably the para-position.

Examples of substituent ($R^a$) of the optionally substituted imino group represented by $Z^{2a}$ and $Z^{Zb}$ include a hydrogen atom, optionally substituted lower ($C_{1-6}$) alkyl {e.g., methyl, ethyl, propyl, isopropyl, butyl, isobutyl, sec-butyl, tert-butyl, pentyl, isopentyl, neopentyl, hexyl, hydroxy-$C_{1-6}$ alkyl (e.g., hydroxyethyl, hydroxypropyl, hydroxybutyl, or the like), halogenated $C_{1-6}$ alkyl (e.g., trifluoromethyl, trifluoroethyl, or the like), cyanated $C_{1-6}$ alkyl (e.g., cyanoethyl, cyanopropyl, or the like), optionally esterified or amidated carboxyl-$C_{1-6}$ alkyl, and the like}, formyl, lower ($C_{2-5}$) alkanoyl (e.g., acetyl, propionyl, butyryl, or the like), lower ($C_{1-5}$) alkylsulfonyl (methylsulfonyl, ethylsulfonyl, etc.), and the like.

Examples of the alkylene group of the "optionally substituted alkylene group" represented by $W^1$ include alkylene chains represented by $-(CH_2)_{k1}-$ (k1 denotes an integer of 1 to 4). Examples of the alkenylene group of the "optionally substituted alkenylene group" represented by $W^1$ include alkenylene chains represented by $-(CH_2)k_2-(CH=CH)-(CH_2)k_3-$ (wherein k2 and k3 are the same or different and denote 0, 1, or 2, and the sum of k2 and k3 is 2 or less). The alkylene groups and alkenylene groups represented by $W^1$ may have substituents at any position (preferably on a carbon atom), and any substituent may be present, provided that it is one that can be bonded to the alkylene chain or alkenylene chain that constitutes the linear chain moiety. Examples thereof include a lower ($C_{1-6}$) alkyl (e.g., methyl, ethyl, propyl,

isopropyl, butyl, isobutyl, sec-butyl, tert-butyl, pentyl, isopentyl, neopentyl, hexyl, or the like), lower ($C_{3-7}$) cycloalkyl (e.g., cyclopropyl, cyclobutyl, cyclopentyl, cyclohexyl, cycloheptyl, or the like), formyl, lower ($C_{2-7}$) alkanoyl (e.g., acetyl, propionyl, butyryl, or the like), optionally esterified phosphono group, optionally esterified or amidated carboxyl group, hydroxyl group, oxo, hydroxyimino group, optionally substituted lower ($C_{1-6}$) alkoxyimino group, and the like, and preferably a lower alkyl having 1 to 6 carbons (preferably a $C_{1-3}$ alkyl), hydroxyl group, oxo, hydroxyimino group, lower ($C_{1-6}$) alkoxyimino group (which may be substituted with a polar group such as hydroxyl group, cyano group, optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like)), and the like.

Examples of the optionally esterified phosphono group are those that are represented by $P(O)(OR^9)(OR^{10})$ (wherein, $R^9$ and $R^{10}$ each denote a hydrogen atom, alkyl group having 1 to 6 carbons, cycloalkyl group having 3 to 7 carbons, or the like; and $R^9$ and $R^{10}$ can be bonded together to form a 5-to 7-membered ring).

In the above formula, examples of the alkyl group having 1 to 6 carbons represented by $R^9$ and $R^{10}$ include methyl, ethyl, propyl, isopropyl, butyl, isobutyl, sec-butyl, tert-butyl, pentyl, isopentyl, neopentyl, hexyl, and the like; and examples of the cycloalkyl having 3 to 7 carbons include cyclopropyl, cyclobutyl, cyclopentyl, cyclohexyl, cycloheptyl, and the like, and preferred groups are a chain lower alkyl having 1 to 6 carbons, and lower alkyls having 1 to 3 carbons are more preferred. $R^9$ and $R^{10}$ may be the same or different, and preferably the same. In addition, when $R^9$ and $R^{10}$ are bonded together to form a 5-to 7-membered ring, $R^9$ and $R^{10}$ are bonded together to form a linear $C_{2-4}$ alkylene side chain represented by $-(CH_2)_2-$, - $(-CH_2)_3-$, or $-(CH_2)_4-$. The side chain may have substituents, and examples of such substituents include a hydroxyl group, a halogen, and the like.

Examples of the ester of the optionally esterified carboxyl group include esters formed by bonding carboxyl group with a cycloalkyl group having 3 to 7 carbons or an alkyl group having 1 to 6 carbons; for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, cyclopentyloxycarbonyl, cyclohexyloxycarbonyl, and the like.

Examples of the amide of the optionally amidated carboxyl group include those produced by bonding carboxyl group with an alkylamino group having 1 to 6 carbons, cycloalkylamino group having 3 to 7 carbons, or 5- to 8-membered cyclic amine (e.g., pyrrolidine, piperidine, morpholine, or the like); for example, carbamoyl, mono-$C_{1-6}$ alkylcarbamoyl, di-$C_{1-6}$ alkylcarbamoyl, cyclopentylaminocarbonyl, cyclohexylaminocarbonyl, pyrrolidinocarbonyl, piperidinocarbonyl, morpholinocarbonyl, thiomorpholinocarbonyl, and the like.

For $Z^2$ , divalent groups are preferred wherein either one of $Z^{2a}$ and $Z^{2b}$ is O, $S(O)_m$ (m is 0, 1, or 2), or -N ($R^a$) - (wherein $R^a$ denotes a hydrogen atom or an optionally substituted lower $C_{1-4}$ alkyl group), and the other is a bond, and W is $-(CH_2)_p-$ (wherein p denotes an integer of 1 to 3), or $Z^2$ is -CH(OH)-. Divalent groups are more preferred wherein either one of $Z^{2a}$ or $Z^{2b}$ is O or $S(O)_m$ (m is 0, 1, or 2) and the other is a bond, and W is $-(CH_2)_p-$(where p denotes an integer of 1 to 3) or $Z^2$ is -CH(OH)-. $Z^2$ is further more preferably $-CH_2-$, -CH(OH)- or $-S(O)_m-CH_2-$(wherein m denotes 0, 1, or 2), and particularly preferably $-S(O)_m-CH_2-$ (m is 0, 1, or 2). When $Z^{2a}$ is bonded to $Z^1$, - $SOCH_2$- is particularly preferred.

$Z^{2a}$ denotes a bond, S, SO, or $SO_2$, among these, SO is preferred, and in such a case, compounds are preferred wherein the steric configuration of the SO is (S).

In the above formula [I], examples of the "optionally substituted amino group wherein the nitrogen atom may be converted to a quaternary ammonium or oxide" represented by $R^2$ include an amino group that may have 1 to 2 substituents, and an amino group having three substituents wherein the nitrogen atom has been converted to a quaternary ammonium. When the number of substituents on the nitrogen atom is 2 or more, these substituents may be the same or different, and when the number of substituents on the nitrogen atom is 3, the amino group may be any type of $-N^+R^pR^pR^p$, $-N^+R^pR^pR^q$, and $-N^+R^pR^qR^r$ (wherein $R^p$, $R^q$, and $R^r$ are each different and denote a hydrogen atom or a substituent). In addition, examples of the counter anion for the amino group wherein the nitrogen atom has been converted to a quaternary ammonium include halogen atom anions (e.g., $Cl^-$, $Br^-$, $I^-$ or the like), as well as anions derived from inorganic acids such as hydrochloric acid, hydrobromic acid, nitric acid, sulfuric acid and phosphoric acid; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid; anions derived from acidic amino acid such as aspartic acid and glutamic acid; and the like, inter alia, $Cl^-$, $Br^-$ and $I^-$ is preferred.

Examples of the substituent of the amino group include:

(1) an optionally substituted alkyl (e.g., $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, preferably a lower ($C_{1-6}$) alkyl or the like);
(2) an optionally substituted cycloalkyl (e.g., $C_{3-8}$ cycloalkyl such as cyclopropyl, cyclobutyl, cyclopentyl, cyclohexyl, cycloheptyl, cyanooctyl, or the like);
(2-1) the above cycloalkyl may contain one heteroatom selected from sulfur atom, oxygen atom, and nitrogen atom, and may form an oxirane, thiolane, aziridine, tetrahydrofuran, tetrahydrothiophene, pyrrolidine, tetrahydropyran, tetrahydrothiopyran, tetrahydrothiopyran, 1-oxide, or piperidine (preferably a 6-membered ring such as tetrahydropyran, tetrahydrothiopyran, piperidine, etc.), and the like, and with respect to the bonding site to the amino group, 3- or 4-position (preferably 4-position) is preferred;

(2-2) in addition, the cycloalkyl can condense with a benzene ring to form an indane (e.g., indan-1-yl, indan-2-yl, or the like), tetrahydronaphthalene (e.g., tetrahydronaphthalen-5-yl, tetrahydronaphthalen-6-yl, etc.), and the like (preferably an indane or the like);

(2-3) in addition, the cycloalkyl can crosslink via a linear atom chain having 1 to 2 carbons to form a crosslinked cyclic hydrocarbon residue such as bicyclo[2.2.1]heptyl, bicyclo[2.2.2]octyl, bicyclo[3.2.1]octyl, bicyclo[3.2.2]nonyl, and the like (preferably a cyclohexyl having a cross-link via a linear atomic chain having 1 to 2 carbons, and more preferably bicyclo[2.2.1]heptyl and the like).

(3) an optionally substituted alkenyl (e.g., alkenyl having 2 to 10 carbons such as allyl, crotyl, 2-pentenyl, 3-hexenyl, and preferably a lower ($C_{2-6}$) alkenyl or the like);

(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);

(5) an optionally substituted aralkyl (e.g., phenyl-$C_{1-4}$ alkyl (e.g., benzyl, phenethyl, or the like);

(6) a formyl or optionally substituted acyl (e.g., an alkanoyl having 2 to 4 carbons (e.g., acetyl, propionyl, butyryl, isobutyryl, or the like), alkyl sulfonyl having 1 to 4 carbons (e.g., methanesulfonyl, ethanesulfonyl, or the like), alkoxycarbonyl having 1 to 4 carbons (e.g., methoxycarbonyl, ethoxycarbonyl, tert-butoxycarbonyl, or the like), or aralkyloxycarbonyl having 7 to 10 carbons (e.g., benzyloxycarbonyl, etc.), or the like);

(7) an optionally substituted aryl (e.g., phenyl, naphthyl, or the like);

(8) an optionally substituted heterocyclic group (e.g., group formed by removing one hydrogen atom from a 5- to 6-membered aromatic heterocyclic ring comprising 1 to 4 of 1 or 2 kinds of heteroatoms selected from nitrogen atom, sulfur atom, and oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, and thiadiazole; a group formed by removing one hydrogen atom from a condensed heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, or the like; or a group formed by removing one hydrogen atom from a 5- to 6-membered non-aromatic heterocyclic ring comprising 1 to 4 of one or two kinds of heteroatoms selected from nitrogen atom, sulfur atom, and oxygen atom, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran tetrahydropyran, or the like; preferably a group formed by removing one hydrogen atom from a 5- to 6-membered non-aromatic heterocycle, or the like; more preferably a group formed by removing one hydrogen atom from a 5- to 6-membered non-aromatic heterocyclic ring comprising one heteroatom such as tetrahydrofuran, piperidine, tetrahydropyran, tetrahydrothiopyran, etc.), and the like. In addition, the substituents of the amino group may be bonded together to form a 5- to 7-membered cyclic amino such as piperidine, piperazine, morpholine, and thiomorpholine.

Examples of the substituent that may be possessed by the (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, (7) optionally substituted aryl, and (8) optionally substituted heterocyclic group include a halogen (e.g., fluorine, chlorine, bromine, iodine, or the like), optionally halogenated lower ($C_{1-4}$) alkyl, lower ($C_{1-4}$) alkyl optionally substituted with a polar group such as hydroxyl group, cyano group, an optionally esterified or amidated carboxyl group (e.g., hydroxy-$C_{1-4}$ alkyl, cyano-$C_{1-4}$ alkyl, carboxyl-$C_{1-4}$ alkyl, $C_{1-4}$ alkoxycarbonyl-$C_{1-4}$ alkyl, carbamoyl-$C_{1-4}$ alkyl, mono-$C_{1-4}$ alkylcarbamoyl-$C_{1-4}$ alkyl, di-$C_{1-4}$ alkylcarbamoyl, di-$C_{1-4}$ alkylcarbamoyl-$C_{1-4}$ alkyl, pyrrolidinocarbonyl-$C_{1-4}$ alkyl, piperidinocarbonyl-$C_{1-4}$ alkyl, morpholinocarbonyl-$C_{1-4}$ alkyl, thiomorpholinocarbonyl-$C_{1-4}$ alkyl, or the like), optionally halogenated $C_{1-4}$ alkoxy (e.g., methoxy, ethoxy, propoxy, butoxy, trifluoromethoxy, trifluoroethoxy, or the like), $C_{1-4}$ alkylenedioxy (e.g., $-O-CH_2-O-$, $-O-CH_2-CH_2-O-$, or the like), formyl, $C_{2-4}$ alkanoyl (e.g., acetyl,, propionyl, or the like), $C_{1-4}$ alkylsulfonyl (e.g., methanesulfonyl, ethanesulfonyl, or the like), phenyl-lower ($C_{1-4}$) alkyl, $C_{3-7}$ cycloalkyl, cyano, nitro, hydroxyl group, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), lower ($C_{1-4}$) alkoxycarbonyl, lower ($C_{7-10}$) aralkyloxycarbonyl, oxo group (preferably a halogen, optionally halogenated lower ($C_{1-4}$) alkyl, optionally halogenated lower ($C_{1-4}$) alkoxy, phenyl-lower ($C_{1-4}$) alkyl, $C_{3-7}$ cycloalkyl, cyano, hydroxyl group, etc.), and the like, and the number of substituents is preferably 1 to 3.

The "optionally substituted amino group wherein the nitrogen atom is converted to a quaternary ammonium or an oxide" represented by $R^2$ in formula (I) above preferably is an amino group having 1 to 3 substituents selected from:

(1) a linear or branched lower ($C_{1-6}$) alkyl having 1 to 3 of halogens, cyano, hydroxyl or $C_{3-7}$ cycloalkyl;

(2) a $C_{5-8}$ cycloalkyl which may have 1 to 3 of halogens, lower ($C_{1-4}$) alkyls that may be halogenated or phenyl-lower ($C_{1-4}$) alkyls, may contain one heteroatom selected from sulfur atom, oxygen atom and nitrogen atom, may be condensed with a benzene ring, and may be crosslinked via a linear atomic chain having 1 to 2 carbons (e.g., cyclopentyl, cyclohexyl, cycloheptyl, cyclooctyl, tetrahydropyranyl, tetrahydrothiopyranyl, piperidinyl, indanyl, tetrahydronaphthalenyl, bicyclo[2.2.1]heptyl each of which may be substituted, and the like);

(3) a phenyl-lower ($C_{1-4}$) alkyl which may have 1 to 3 of halogen, lower ($C_{1-4}$) alkyl that may be halogenated or lower ($C_{1-4}$) alkoxy that may be halogenated;

(4) a phenyl that may have 1 to 3 of halogen, optionally halogenated lower ($C_{1-4}$) alkyl, or optionally halogenated lower ($C_{1-4}$) alkoxy; and

(5) 5- to 6-membered aromatic heterocyclic group that may have 1 to 3 of halogen, optionally halogenated lower ($C_{1-4}$) alkyl, optionally halogenated lower ($C_{1-4}$) alkoxy, optionally halogenated lower ($C_{1-4}$) alkoxy-lower ($C_{1-4}$) alkoxy, phenyl-lower ($C_{1-4}$) alkyl, cyano, or hydroxyl.

The "nitrogen-containing heterocycle" of the "optionally substituted nitrogen-containing heterocyclic group which may comprise sulfur atom or oxygen atom as ring constituent atoms and wherein the nitrogen atom may be converted into a quaternary ammonium or oxide" represented by $R^2$ in formula (I) above includes a 5- to 6-membered aromatic heterocycle comprising 1 to 4 of 1 or 2 kinds of heteroatoms selected from nitrogen atom, sulfur atom, and oxygen atom such as pyrrole, imidazole, pyrazole, thiazole, oxazole, isothiazole, isoxazole, tetrazole, pyridine, pyrazine, pyrimidine, pyridazine, triazole, oxadiazole, thiadiazole; a condensed aromatic heterocycle such as benzofuran, indole, benzothiophene, benzoxazole, benzothiazole, indazole, benzimidazole, quinoline, isoquinoline, quinoxaline, phthalazine, quinazoline, cinnoline, imidazopyridine; and a 5- to 8-membered non-aromatic heterocycle that may have 1 to 3 of 1 or 2 kinds of heteroatoms selected from nitrogen atom, sulfur atom and oxygen atom in addition to one nitrogen atom such as pyrrolidine, pyrroline, imidazolidine, imidazoline, pyrazolidine, pyrazoline, piperidine, piperazine, oxazine, oxadiazine, thiazine, thiadiazine, morpholine, thiomorpholine, azacycloheptane, azacyclooctane (azocaine), and the like, and these nitrogen-containing heterocycles may crosslink via a linear atomic chain having 1 to 2 carbons to form crosslinked cyclic nitrogen-containing heterocycles such as azabicyclo[2.2.1]heptane, azabicyclo[2.2.2]octane (quinuclidine), and the like (preferably piperidine having crosslinkage via a linear atomic chain of 1 to 2 carbons).

Among the specific examples of the above nitrogen-containing heterocycle, pyridine, pyridazine, pyrazole, imidazole, triazole, tetrazole, imidazopyridine, pyrrolidine, piperidine, piperazine, morpholine, thiomorpholine and azabicyclo[2.2.2]octane (preferably, pyridine, imidazole, triazole, imidazopyridine, pyrrolidine, piperidine and morpholine).

The nitrogen atom of the "nitrogen-containing heterocycle" may be converted to a quaternary ammonium or may be oxidized. When the nitrogen atom of the "nitrogen-containing heterocycle" is converted to a quaternary ammonium, examples of the counter anion for the "nitrogen-containing heterocyclic group wherein the nitrogen atom is converted to a quaternary ammonium" include halogen atom anions (e.g., $Cl^-$, $Br^-$, and $I^-$), as well as anions derived from inorganic acid such as hydrochloric acid, hydrobromic acid, nitric acid, sulfuric acid and phosphoric acid; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid; and anions derived from acidic amino acid such as aspartic acid and glutamic acid, and the like, with $Cl^-$, $Br^-$, and $I^-$ being preferred among them.

The "nitrogen-containing heterocyclic group" may be bonded to the divalent group represented by $Z^2$ via either a nitrogen atom or carbon atom, and may be bonded via the ring constituent carbon atom like 2-pyridyl, 3-pyridyl, 2-piperidinyl and the like, and also may be bonded via the ring constituent nitrogen atom, as with:

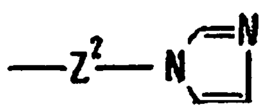,
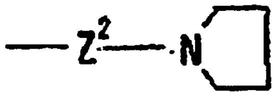,
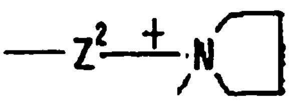,

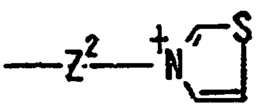,
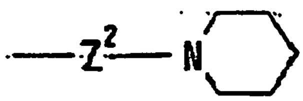,
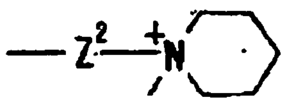,

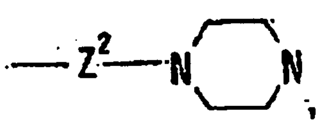,
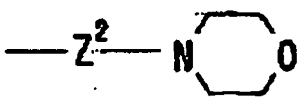,
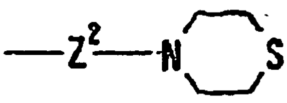,

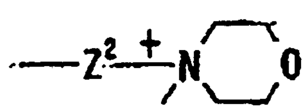, 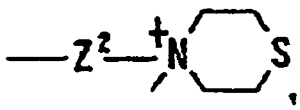, 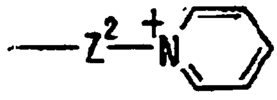,

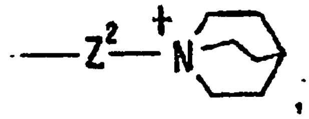, 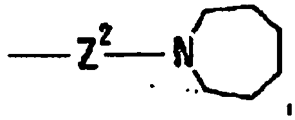, 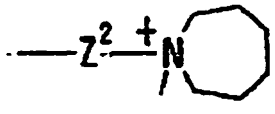,

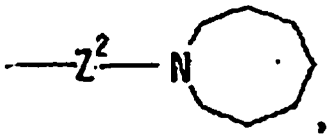, 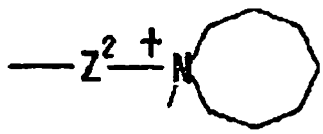

and the like.

Examples of the substituent that the "nitrogen-containing heterocycle" may have, include a halogen (e.g., fluorine, chlorine, bromine, iodine, or the like), optionally substituted lower ($C_{1-4}$) alkyl, optionally substituted lower ($C_{1-4}$) alkoxy, optionally substituted phenyl, optionally substituted mono- or di-phenyl-lower ($C_{1-4}$) alkyl, optionally substituted $C_{3-7}$ cycloalkyl, cyano, nitro, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), lower ($C_{1-4}$) alkoxycarbonyl, formyl, lower ($C_{2-4}$) alkanoyl, lower ($C_{1-4}$) alkylsulfonyl, optionally substituted heterocyclic group (e.g., group formed by removing one hydrogen atom from a 5- to 6-membered aromatic heterocyclic ring comprising 1 to 4 of 1 or 2 kinds of heteroatoms selected from nitrogen atom, sulfur atom and oxygen atom, such as a furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, or the like; or a group formed by removing one hydrogen atom from a condensed aromatic heterocyclic group containing 1 to 4 of 1 to 2 kinds of heteroatoms selected from nitrogen atom, sulfur atom and oxygen atom, such as benzofuran, indole, benzothiophene, benzoxazole, benzothiazole, indazole, benzimidazole, quinoline, isoquinoline, quinoxaline, phthalazine, quinazoline, cinnoline, imidazopyridine, or the like; or a group formed by removing one hydrogen atom from a 5- to 6-membered non-aromatic heterocyclic ring comprising 1 to 4 of one or two kinds of heteroatoms selected from nitrogen atom, sulfur atom and oxygen atom, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, or the like, and the number of the substituents is preferably 1 to 3. In addition, the nitrogen atom of the "nitrogen-containing heterocycle" may be oxidized.

Examples of the substituent that may be possessed by the "optionally substituted lower ($C_{1-4}$) alkyl", "optionally substituted lower ($C_{1-4}$) alkoxy", "optionally substituted phenyl", "optionally substituted mono- or di-phenyl-lower ($C_{1-4}$) alkyl", "optionally substituted $C_{3-7}$ cycloalkyl" and "optionally substituted heterocyclic group" as the substituent that the "nitrogen-containing heterocycle" may have, include a halogen (e.g., fluorine, chlorine, bromine, iodine, or the like), optionally halogenated lower ($C_{1-4}$) alkyl, lower ($C_{1-4}$) alkyl optionally substituted with a polar group such as hydroxyl, cyano and carboxyl group that may be esterified or amidated (e.g., hydroxy-$C_{1-4}$ alkyl, cyano-$C_{1-4}$ alkyl, carboxyl-$C_{1-4}$ alkyl, $C_{1-4}$ alkoxycarbonyl-$C_{1-4}$ alkyl, carbamoyl-$C_{1-4}$ alkyl, mono-$C_{1-4}$ alkylcarbamoyl-$C_{1-4}$ alkyl, di-$C_{1-4}$ alkylcarbamoyl-$C_{1-4}$ alkyl, pyrrolidinocarbonyl-$C_{1-4}$ alkyl, piperidinocarbonyl-$C_{1-4}$ alkyl, morpholinocarbonyl-$C_{1-4}$ alkyl, thiomorpholinocarbonyl-$C_{1-4}$ alkyl or the like), lower ($C_{3-10}$) cycloalkyl, lower ($C_{3-10}$) cycloalkenyl, optionally halogenated $C_{1-4}$ alkoxy (e.g., methoxy, ethoxy, trifluoromethoxy, trifluoroethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, or the like), $C_{1-3}$ alkylenedioxy (e.g., methylenedioxy, ethylenedioxy, or the like), cyano, nitro, hydroxyl group, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), lower ($C_{1-4}$) alkoxycarbonyl, and the like, where the number of the substituents is preferably 1 to 3.

In the above formula [I], preferred examples of the substituent that may be possessed by the "nitrogen-containing

heterocycle" of the "optionally substituted nitrogen-containing heterocyclic group which may comprise sulfur atoms or oxygen atoms as ring constituent atoms and wherein the nitrogen atom may be converted into a quaternary ammonium or oxide" are (1) a halogen, (2) a cyano, (3) a hydroxyl group, (4) a carboxyl group, (5) a carbamoyl group, (6) a lower ($C_{1-4}$) alkoxycarbonyl, (7) a lower ($C_{1-4}$) alkylcarbamoyl or 5- to 6-membered cyclic amino (piperidino, morpholino or the like)-carbonyl, (8) a lower ($C_{1-4}$) alkyl optionally substituted with a halogen, hydroxyl group, cyano group, lower ($C_{1-4}$) alkoxy, or optionally esterified or amidated carboxyl group, (9) a lower ($C_{1-4}$) alkoxy optionally substituted with halogen, hydroxyl group or lower ($C_{1-4}$) alkoxy, (10) a phenyl optionally substituted with a halogen, lower ($C_{1-4}$) alkyl, hydroxyl group, lower ($C_{1-4}$) alkoxy, or $C_{1-3}$ alkylenedioxy, (11) mono- or diphenyl-lower ($C_{1-4}$) alkyl optionally substituted with a halogen, lower ($C_{1-4}$) alkyl, hydroxyl group, lower ($C_{1-4}$) alkoxy, or ($C_{1-3}$) alkylenedioxy, and (12) a group formed by removing one hydrogen atom from a 5- to 6-membered aromatic heterocycle such as furan, thiophene, pyrrole, and pyridine.

In the above formula [I], examples of the "optionally substituted hydrocarbon group" represented by $R^5$ and $R^6$ in the "group represented by the formula:

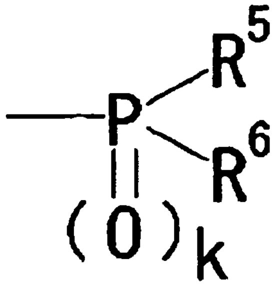

wherein, k denotes 0 or 1, and when k is 0, the phosphorus atom can form a phosphonium salt; $R^5$ and $R^6$ each denote an optionally substituted hydrocarbon group, optionally substituted hydroxyl group, or optionally substituted amino group (preferably an optionally substituted hydrocarbon group or optionally substituted amino group, more preferably an optionally substituted hydrocarbon group); and $R^5$ and $R^6$ can be bonded together to form a cyclic group along with an adjacent phosphorus atom" represented by $R^2$ include the following:

(1) an optionally substituted alkyl (e.g., $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and preferably a lower ($C_{1-6}$) alkyl or the like);
(2) an optionally substituted cycloalkyl (e.g., $C_{3-7}$ cycloalkyl such as cyclopropyl, cyclobutyl, cyclopentyl, cyclohexyl, cycloheptyl, or the like);
(3) an optionally substituted alkenyl (e.g., alkenyl having 2 to 10 carbons such as allyl, crotyl, 2-pentenyl, 3-hexenyl, and preferably a lower ($C_{2-6}$) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) an alkynyl that may be substituted (e.g., alkynyl having 2 to 10 carbons such as ethynyl, 1-propynyl, 2-propynyl, 1-butynyl, 2-pentynyl, 3-hexynyl, and preferably a lower ($C_{2-6}$) alkynyl or the like);
(6) an optionally substituted aralkyl (e.g., phenyl-$C_{1-4}$ alkyl (e.g., benzyl, phenethyl, etc.), or the like);
(7) an optionally substituted aryl (e.g., phenyl, naphthyl, etc.), and the like, and examples of the substituent that may be possessed by the (1) optionally substituted alkyl, (2) optionally substituted cycloalkyl, (3) optionally substituted alkenyl, (4) optionally substituted cycloalkenyl, (5) optionally substituted alkynyl, (6) optionally substituted aralkyl, and (7) optionally substituted aryl mentioned above include a halogen (e.g., fluorine, chlorine, bromine, iodine, or the like), nitro, cyano, hydroxyl group, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), optionally halogenated $C_{1-4}$ alkyl (e.g., trifluoromethyl, methyl, ethyl, or the like), optionally halogenated $C_{1-4}$ alkoxy (e.g., methoxy, ethoxy, trifluoromethoxy, trifluoroethoxy, or the like), formyl, $C_{2-4}$ alkanoyl (e.g., acetyl, propionyl, or the like), and $C_{1-4}$ alkylsulfonyl (e.g., methanesulfonyl, ethanesulfonyl, etc.), and the like, where the number of substituents is preferably 1 to 3.

Examples of the "optionally substituted hydroxyl group" represented by $R^5$ and $R^6$ include hydroxyl groups that may have:

(1) an optionally substituted alkyl (e.g., $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, preferably a lower ($C_{1-6}$) alkyl or the like);
(2) an optionally substituted cycloalkyl (e.g., $C_{3-7}$ cycloalkyl such as cyclopropyl, cyclobutyl, cyclopentyl, cyclohexyl, cycloheptyl, or the like),
(3) an optionally substituted alkenyl (e.g., alkenyl having 2 to 10 carbons such as allyl, crotyl, 2-pentenyl, 3-hexenyl, and preferably a lower ($C_{2-6}$) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) an optionally substituted aralkyl (e.g., phenyl-$C_{1-4}$ alkyl (e.g., benzyl or phenethyl, etc.), or the like);
(6) a formyl or an optionally substituted acyl (e.g., an alkanoyl having 2 to 4 carbons (e.g., acetyl, propionyl, butyryl, isobutyryl, or the like), or an alkylsulfonyl having 1 to 4 carbons (e.g., methanesulfonyl, ethanesulfonyl, etc.) or the like);
(7) an optionally substituted aryl (e.g., phenyl, naphthyl, etc.), and the like.

Examples of the substituent that may be possessed by the (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, and (7) optionally substituted aryl mentioned above include a halogen (e.g., fluorine, chlorine, bromine, iodine, or the like), nitro, cyano, hydroxyl group, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), optionally halogenated $C_{1-4}$ alkyl (e.g., trifluoromethyl, methyl, ethyl, or the like), optionally halogenated $C_{1-4}$ alkoxy (e.g., methoxy, ethoxy, trifluoromethoxy, trifluoroethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, where the number of substituents is preferably 1 to 3.

In the above formula, $R^5$ and $R^6$ may be bonded together along with an adjacent phosphorus atom to form a cyclic group (preferably a 5- to 7-membered ring). The cyclic group may have substituents, and examples of the substituents include a halogen, (e.g., fluorine, chlorine, bromine, iodine, or the like), nitro, cyano, hydroxyl, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, or di-$C_{1-4}$ alkylcarbamoyl), optionally halogenated $C_{1-4}$ alkyl (e.g., trifluoromethyl, methyl, ethyl, or the like), optionally halogenated $C_{1-4}$ alkoxy (e.g., methoxy, ethoxy, trifluoromethoxy, trifluoroethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, where the number of substituents is preferably 1 to 3.

Examples of the counter anion when the phosphorus atom forms a phosphonium salt in formula (I) above include halogen atom anions (e.g., $Cl^-$, $Br^-$, and $I^-$) as well as anions derived from inorganic acid such as hydrochloric acid, hydrobromic acid, nitric acid, sulfuric acid and phosphoric acid; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid; anions derived from acidic amino acid such as aspartic acid and glutamic acid; and the like, with $Cl^-$, $Br^-$, and $I^-$ being preferred.

Examples of the optionally substituted amino group represented by $R^5$ and $R^6$ include amino groups that may have 1 or 2 of:

(1) an optionally substituted alkyl (e.g., $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, preferably a lower ($C_{1-6}$) alkyl or the like);
(2) an optionally substituted cycloalkyl (e.g., $C_{3-7}$ cycloalkyl such as cyclopropyl, cyclobutyl, cyclopentyl, cyclohexyl, cycloheptyl, or the like),
(3) an optionally substituted alkenyl (e.g., alkenyl having 2 to 10 carbons such as allyl, crotyl, 2-pentenyl, 3-hexenyl, and preferably a lower ($C_{2-6}$) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) formyl or an optionally substituted acyl (e.g., an alkanoyl having 2 to 4 carbons (e.g., acetyl, propionyl, butyryl, isobutyryl, or the like), or an alkylsulfonyl having 1 to 4 carbons (e.g., methanesulfonyl, ethanesulfonyl, etc.) or the like); and
(6) an optionally substituted aryl (e.g., phenyl, naphthyl, etc.), and the like.

Examples of the substituent that may be possessed by the (1) optionally substituted alkyl, (2) optionally substituted cycloalkyl, (3) optionally substituted alkenyl, (4) optionally substituted cycloalkenyl, (5) optionally substituted

acyl, and (6) optionally substituted aryl mentioned above include a halogen (e.g., fluorine, chlorine, bromine, iodine, or the like), nitro, cyano, hydroxyl group, optionally substituted thiol group (e.g., thiol, $C_{1-4}$ alkylthio, or the like), optionally substituted amino group (e.g., amino, mono-$C_{1-4}$ alkylamino, di-$C_{1-4}$ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, $C_{1-4}$ alkoxycarbonyl, carbamoyl, mono-$C_{1-4}$ alkylcarbamoyl, di-$C_{1-4}$ alkylcarbamoyl, or the like), optionally halogenated $C_{1-4}$ alkyl (e.g., trifluoromethyl, methyl, ethyl, or the like), optionally halogenated $C_{1-4}$ alkoxy (e.g., methoxy, ethoxy, trifluoromethoxy, trifluoroethoxy, or the like), formyl, $C_{2-4}$ alkanoyl (e.g., acetyl, propionyl, or the like), $C_{1-4}$ alkylsulfonyl (e.g., methanesulfonyl, ethanesulfonyl, etc.), and the like, where the number of substituents is preferably 1 to 3.

Examples of the substituent in the "optionally substituted amidino group" and "optionally substituted guanidino group" represented by $R^2$ are the same as those in the "optionally substituted amino group wherein the nitrogen atom may be converted to a quaternary ammonium or oxide" represented by $R^2$ above.

$R^2$ is preferably (1) an optionally substituted amino group wherein the nitrogen atom may be converted to a quaternary ammonium or oxide, (2) an optionally substituted nitrogen-containing heterocyclic group which may comprise a sulfur atom or oxygen atom as a ring constituent atom, and wherein the nitrogen atom may be converted to a quaternary ammonium or oxide, (3) an optionally substituted amidino group, or (4) an optionally substituted guanidino group, and $R^2$ is more preferably an optionally substituted amino group wherein the nitrogen atom may be converted to a quaternary ammonium or oxide, an optionally substituted nitrogen-containing heterocyclic group which may comprise a sulfur atom or oxygen atom as a ring constituent atom, and wherein the nitrogen atom may be converted to an oxide, and particularly preferred is an optionally substituted amino group or an optionally substituted nitrogen-containing heterocyclic group which may comprise an oxygen atom or sulfur atom as a ring constituent atom.

$R^2$ is furthermore preferably a group represented by the formula -NRR" or -N+RR'R" (wherein, R, R', and R" each denote an optionally substituted aliphatic hydrocarbon group (aliphatic chain hydrocarbon group or aliphatic cyclic hydrocarbon group) or an optionally substituted alicyclic (non-aromatic) heterocyclic group), or an optionally substituted nitrogen-containing aromatic heterocyclic group wherein the nitrogen atom may be oxidized.

Examples of the "optionally substituted aliphatic hydrocarbon group" and "optionally substituted alicyclic heterocyclic group" represented by R, R', and R " in the above formula are the same groups as the "optionally substituted aliphatic hydrocarbon groups (e.g., alkyl, cycloalkyl, alkenyl, cycloalkenyl, or the like, each of which may be substituted)" and the "optionally substituted alicyclic heterocyclic groups (e.g., optionally substituted 5- to 6-membered non-aromatic heterocycles and the like)" exemplified for the substituents that the "optionally substituted amino group" represented by substituent $R^2$ may have.

Among these groups, optionally substituted chain hydrocarbon groups (e.g., optionally substituted alkyl, alkenyl, and the like) are preferred for R and R', and optionally substituted $C_{1-6}$ alkyl groups are more preferred, and further an optionally substituted methyl group is particularly preferred.

R" is preferably an optionally substituted alicyclic hydrocarbon group (preferably, an optionally substituted $C_{3-8}$ cycloalkyl group; more preferably an optionally substituted cyclohexyl) or an optionally substituted alicyclic heterocyclic group (preferably an optionally substituted saturated alicyclic heterocyclic group (preferably a 6-membered cyclic group); more preferably an optionally substituted tetrahydropyranyl, optionally substituted tetrahydrothiopyranyl, or optionally substituted piperidyl; and particularly preferably an optionally substituted tetrahydropyranyl).

In addition, among the pyridine, imidazole, triazole, and imidazopyridine that are exemplified for the preferred "nitrogen-containing aromatic heterocyclic groups" of the "optionally substituted nitrogen-containing aromatic heterocyclic group wherein the nitrogen atom may be oxidized" represented by $R^2$, an imidazole or triazole is particularly preferable.

As for the "optionally substituted amino group wherein the nitrogen atom may be converted to a quaternary ammonium or oxide" and the like represented by $R^{2'}$ and $R^{2''}$, the same as in the corresponding groups of $R^2$ mentioned above may be exemplified.

As for the "optionally substituted hydrocarbon group", "optionally substituted $C_{1-6}$ alkyl" and the like in the substituent represented by $R^4$ for the imino group of Y and the substituent for the imino group of Y', the same as in the corresponding groups of $R^o$ mentioned above may be exemplified.

The same as in the corresponding groups of $W^1$ mentioned above may be exemplified for the "optionally substituted alkylene chain" of $W^2$.

As the compounds represented by formula (I), the compounds below are preferred.

8-[4-(2-Butoxyethoxy)phenyl]-N-[4-[[N-methyl-N-(tetahydropyran-4-yl)amino]methyl]phenyl]-3,4-dihydro-2H-1-benzoxocin-5-carboxamide;

8-[4-(2-Butoxyethoxy)phenyl]-N-[4-[[N-methyl-N-(tetahydropyran-4-yl)amino]methyl]phenyl]-1,2,3,4-tetrahydro-1-benzazocine-5-carboxamide;

8-[4-(2-Butoxyethoxy)phenyl]-1-propyl-N-[4-[[[1-propylimidazol-5-yl]methyl]sulfanyl]phenyl]-1,2,3,4-tetrahydro-l-benzazocine-5-carboxamide;

8-[4-(2-Butoxyethoxy)phenyl]-1-propyl-N-[4-[[[1-propylimidazol-5-yl]methyl]sulfinyl]phenyl]-1,2,3,4-tetrahydro-1-benzazocine-5-carboxamide;
8-[4-(2-Butoxyethoxy)phenyl]-1-propyl-N-[4-[[[1-propylimidazol-5-yl]methyl]sulfonyl]phenyl]-1,2,3,4-tetrahydro-l-benzazocine-5-carboxamide;
8-[4-(2-Butoxyethoxy)phenyl]-1-isobutyl-N-[4-[[[1-propylimidazol-5-yl]methyl]sulfanyl]phenyl]-1,2,3,4-tetrahydro-1-benzazocine-5-carboxamide;
8-[4-(2-Butoxyethoxy)phenyl]-1-isobutyl-N-[4-[[[1-propylimidazol-5-yl]methyl]sulfinyl]phenyl]-1,2,3,4-tetrahydro-1-benzazocine-5-carboxamide:
8-[4-(2-Butoxyethoxy)phenyl]-1-isobutyl-N-[4-[[[1-propylimidazol-5-yl]methyl]sulfonyl]phenyl]-1,2,3,4-tetrahydro-1-benzazocine-5-carboxamide;
8-[4-(2-Butoxyethoxy)phenyl]-1-isobutyl-N-[4-[[N-methyl-N-(tetrahydropyran-4-yl)amino]methyl]phenyl]-1,2,3,4-tetrahydro-1-benzazocine-5-carboxamide;
(S)-8-[4-(2-Butoxyethoxy)phenyl]-1-isobutyl-N-[4-[[(1-propyl-1H-imidazol-5-yl)methyl]sulfinyl]phenyl]-1,2,3,4-tetrahydro-1-benzazocine-5-carboxamide methanesulfonate;
(S)-8-[4-(2-Butoxyethoxy)phenyl]-1-propyl-N-[4-[[(1-propyl-1H-imidazol-5-yl)methyl]sulfinyl]phenyl]-1,2,3,4-tetrahydro-1-benzazocine-5-carboxamide methanesulfonate;
(S)-1-isobutyl-8-[4- (2-propoxyethoxy)phenyl]-N-[4-[[(1-propyl-1H-imidazol-5-yl)methyl]sulfinyl]phenyl]-1,2,3,4-tetrahydro-1-benzazocine-5-carboxamide;
(S)-8-[4-(2-Butoxyethoxy)phenyl]-1-[(1-methyl-1H-pyrazol-4-yl)methyl]-N-[4-[[(1-propyl-1H-imidazol-5-yl)methyl]sulfinyl]phenyl]-1,2,3,4-tetrahydro-1-benzazocine-5-carboxamide; and
(S)-8-[4-(2-butoxyethoxy)phenyl]-1-isobutyl-N-[4-[[(4-propyl-4H-1,2,4-triazol-3-yl)methyl]sulfinyl]phenyl]-1,2,3,4-tetrahydro-1-benzazocine-5-carboxamide.

Pharmacologically acceptable salts are preferred for the salts of the compounds represented by formula (I), and examples include salts with inorganic bases, salts with organic bases, salts with inorganic acids, salts with organic acids, salts with basic or acidic amino acids, and the like. Suitable examples of salts with inorganic bases include alkali metal salts such as sodium salts and potassium salts; alkaline earth metal salts such as calcium salts and magnesium salts; aluminum salts and ammonium salts, and the like. Suitable examples of salts with organic bases include a salt with trimethylamine, triethylamine, pyridine, picoline, ethanolamine, diethanolamine, triethanolamine, dicyclohexylamine, N,N'-dibenzylethylenediamine, and the like. Suitable examples of salts with inorganic acids include salts with hydrochloric acid, hydrobromic acid, nitric acid, sulfuric acid, phosphoric acid, and the like. Suitable examples of salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like. Suitable examples of salts with basic amino acids include salts with arginine, lysine, ornithine, and the like, and suitable examples of salts with acidic amino acids include salts with aspartic acid, glutamic acid, and the like.

The compounds represented by formula (I) above or salts thereof can be produced according to methods known per se, such as those described in JP-A 2003-335776 and JP-A 08-73476, or analogous methods thereto.

The pharmaceutical composition of the present invention can be manufactured by a per se known method. That is, all the components other than the medical active ingredient are warmed with a hot-water bath or another method, and each component is mixed uniformly. Then, the medical active ingredient is added to this uniform mixed solution, which is mixed thoroughly to give the pharmaceutical composition. In addition, the composition can be filled into a capsule according to a conventional method.

The preparation of the present invention is an oral preparation which comprises enclosing the semisolid or liquid pharmaceutical composition containing two or more surfactants having different molecular weights described above, and may be a soft capsule, hard capsule, stick pack, drink, or liquid to be weighed out at the time of use. These preparations can be manufactured by the methods described in the general preparation principles of the $14^{th}$ revised version of Japanese Pharmacopoeia.

The semisolid or liquid pharmaceutical composition comprising two or more surfactants having different molecular weights of the present invention has an ability to form or maintain a stable microemulsion, and consequently, when the preparation of the present invention is administered orally, a stable microemulsion in which fine particles comprising the medicinal active ingredient are dispersed, is formed or maintained in the digestive tract. Therefore, the absorbability of the medicinal active ingredient, particularly hardly water-soluble active ingredient, from the digestive tract has dramatically improved, and thus its bioavailability is increased. In addition, the whole pharmaceutical composition of the present invention doesn't always have to form or maintain microemulsion.

Since the compound or a salt thereof represented by formula (I) above has a superior CCR antagonistic action, particularly CCR5 and/or CCR2 antagonistic action, inter alia potent CCR5 antagonistic action, it can be used for HIV infection in human, for example, for prevention or treatment of AIDS and for prevention or treatment of various other diseases. In addition, the compound represented by formula (I) above or a salt thereof is low toxic and can be safely used.

For example, the pharmaceutical composition comprising the compound represented by formula (I) above or a salt thereof can be used as a CCR5 antagonist, for example, as a prophylactic or therapeutic agent for AIDS and

inhibitor for progression of pathology of AIDS. Moreover, the pharmaceutical composition that comprises the compound represented by formula (I) above or a salt thereof can be used as a prophylactic or therapeutic agent for various diseases such as a prophylactic or therapeutic agent for transplant graft-versus-host disease and/or rejection reactions, and a prophylactic or therapeutic agent for chronic rheumatoid arthritis, autoimmune disease, allergic diseases, ischemic brain cell injury, myocardial infarct, chronic nephritis, and arterial sclerosis.

Examples of the object disease for the preventive or therapeutic agent of the present invention include transplant rejection reactions (post-transplant rejection reactions, post-transplant erythrocytosis/hypertension/organ injury/vascular thickening, graft-versus-host reaction, and the like), rigid myelitis and other arthritic bone diseases (chronic rheumatoid arthritis, arthritis deformans, rheumatoid myelitis, osteoporosis, cellular or other hyperplasia, bone fracture, bone refracture, osteomalasia, bone Piaget's disease, osteomyelitis, osteoarthritis of the knee, joint tissue destruction in similar diseases, and the like), autoimmune diseases (collagenosis, systemic erythematodes, pachydermia, polyarteritis nodosa, myasthenia gravis, multiple sclerosis, and the like), allergic diseases (allergic rhinitis, conjunctivitis, digestive tract allergies, pollinosis, anaphylaxy, atopic dermatitis, bronchial asthma, and the like), inflammatory bowel disease (ulcerative colitis, Crohn's disease, gastritis, gastric ulcer, stomach cancer, postoperative stomach injury, indigestion, esophageal ulcer, pancreatitis, colonic polyp, gallstones, hemorrhoids, digestive illnesses, localized ileitis, and the like), inflammatory diseases (retinopathy, inflammation subsequent to surgery or injury, relief of swelling, pharyngitis, cystitis, meningitis, inflammatory eye diseases, and the like), respiratory diseases (common cold, pneumonia, asthma, pulmonary hypertension, pulmonary thrombus, pulmonary embolism, pulmonary sarcoidosis, pulmonary tuberculosis, interstitial pneumonitis, pulmonary silicosis, adult respiratory distress syndrome, chronic obstructive pulmonary disease, and the like), infectious diseases (viral infections from cytomegalovirus, influenza virus, herpes virus, and the like, rickettsial infections, bacterial infections, sexually transmitted diseases, Pneumocystis carinii pneumonia, Helicobacter pylori infection, systemic fungal infection, tuberculosis, aggressive Staphylococcus infection, critical viral encephalitis, acute bacterial meningitis, AIDS encephalitis, toxemia, sepsis, critical sepsis, toxemic shock, endotoxic shock, toxic shock syndrome, and the like), cancer and accompanying cachexia, cancer metastasis (urinary bladder cancer, breast cancer, cervical cancer, ovarian cancer, chronic lymphatic leukemia, chronic myeloid leukemia, colon cancer, rectal cancer, colonic cancer, multiple myeloma, acute myeloma, prostate cancer, lung cancer, stomach cancer, Hodgkin's disease, acute melanoma, acute lymphoma, and the like), non-Hodgkin's lymphoma, non-small cell lung cancer, acute melanoma, degenerative neurological diseases (Alzheimer's disease, Parkinson's disease, amyotrophic lateral sclerosis (ALS), Huntington's disease, diabetic neurological impairment, Creutzfeldt-Jacob disease, and the like), neurological diseases (depression, epilepsy, alcohol dependency, and the like), schizophrenia, arterial function insufficiency, central nervous impairment (symptoms and complications resulting from cerebral hemorrhage or cerebral infarct, external head injury, spinal cord injury, cerebral edema, cognitive function impairment, cognitive function abnormalities, autonomic nervous function impairment, autonomic nervous function abnormality, and the like), central nervous injury (external head injury, spinal cord injury, whiplash, and the like), vascular dementia (multiple infarct dementia, Binswanger's disease, and the like), cerebrovascular damage (asymptomatic cerebrovascular damage, transient cerebral ischemic attack, apoplexy, cerebral vascular dementia, hypertensive encephalopathy, and the like), recurrence of cerebral vascular damage and attendant disease (neurologic symptoms, psychological symptoms, subjective symptoms, impairment of daily activity, and the like), cerebrovascular dementia, post-cerebrovascular-infarct central nervous impairment, cerebrocirculatory injury or abnormality, loss of renal circulation self-regulatory capacity, blood-brain barrier injury, anxiety, unstable angina pectoris and other acute coronary arteriopathic syndromes, mental malaise, amnesia, trigeminal neuralgia, ear, nose, and throat diseases (Meniere's syndrome, tinnitis, dysgeusia, vertigo, disorder of balance, difficulty swallowing, and the like), migraine, chronic pain, skin disorder (keloid, vascular edema, psoriasis, and the like), occlusive arteriosclerosis, occlusive thromboangitis, peripheral arterial occlusion, post-ischemic reperfusion injury, Raynaud's syndrome, Buerger's syndrome, myocarditis, myocardial ischemia, myocardial infarct, post-myocardial-infarct progressive cardiac insufficiency, myocardosis, cardiomegaly, chronic cardiac insufficiency including acute cardiac insufficiency and stasis, stenocardia, arrhythmia, tachycardia, abnormal diurnal blood pressure fluctuation, blood or corpuscular component abnormalities (platelet hypercoagulation, abnormal erythrocyte plasticity, leukocyte adhesion stimulation, blood hyperviscosity, erythrocytosis, vascular purpura, autoimmune hemolytic anemia, disseminated intravascular coagulation syndrome, multiple myeloma, and the like), arterial sclerosis including atheroma (aneurysm, coronary arteriosclerosis, cerebral arteriosclerosis, peripheral arterial sclerosis, and the like), post-bypass vascular reocclusion or restenosis, post-intervention (transdermal coronary arterioplasty, stent placement, coronary arterial endoscopy, vascular ultrasound, coronary perfusion thrombolysis, and the like) vascular stenosis, occlusion and organ injury, generation or hyperfunction of vasculotrophic substances or blood clotting substances (endoserine, thromboxane A2, and the like), neovascularization (including abnormal vasculature formation in capillary network dystrophy at the outer membrane of arteriosclerotic lesions), thrombosis, fatty deposition stimulation, eye diseases (glaucoma, ocular hypertension, and the like), hypertension, hypertensive tinnitis, dialysis hypotension, endothelial cell and organ injury, endocrine diseases (Addison's disease, Cushing's syndrome, melanocytoma, primary hyperaldosteronism, nephritis, kidney diseases (nephritis, glomerulonephritis, glomerulosclerosis, renal insufficiency, thrombotic microangiopathy, diabetic neuropathy, and the like), glucose

tolerance abnormalities, liver disease (hepatitis including chronic hepatitis, cirrhosis of the liver, and the like), interstitial hepatopathy, chronic pancreatitis, portal hypertension, obesity, male infertility, gynecological diseases (climacteric disorder, gestational toxicosis, endometriosis, hysteromyoma, fibroid, ovary disease, breast disease, and the like), breast tumor, chronic fatigue syndrome, prostatomegaly, Behcet's disease, Hodgkin's disease, lacunar infarct, consciousness disorder, psoriasis, diseases resulting from environmental or occupational factors (radiation injury, ultraviolet/infrared/ laser light injury, mountain sickness, and the like), and claudicatio intermittens.

The dosage of the pharmaceutical composition comprising the compound represented by formula (I) above or a salt thereof of the present invention can be selected appropriately depending on the administration subject, the age and body weight of the administration subject, symptoms, administration time, administration method, and dosage form. The dose to specific patients is to be determined in consideration of age, body weight, general physical condition, sex, food, administration time, administration method, excretion rate, and extent of the disease at the time of patient treatment, as well as other factors.

When the above pharmaceutical composition is to be used as a prophylactic or therapeutic agent for AIDS and inhibitor for progression of pathology of AIDS, the dosage differs depending on the patient condition, body weight, and administration method, and for oral administration, it is about 5 to 1000 mg, preferably about 10 to 600 mg, more preferably about 10 to 300 mg, and particularly preferably about 15 to 150 mg in terms of active ingredient (compound represented by formula (I)) per adult (body weight 50 kg), and it is administered in a single dose or in 2 to 3 divided doses per day.

When the pharmaceutical composition comprising the compound represented by formula (I) above or a salt thereof is to be used as a prophylactic or therapeutic agent for graft-versus-host disease and/or rejection reaction in cases of organ transplantation such as the heart, kidney, liver, and bone marrow, it is administered from three days before transplantation, and continuously administered after transplantation. The daily dosage of the pharmaceutical composition of the present invention will differ depending on the patient condition, body weight, and administration method, and for oral administration, it is about 5 to 1000 mg, preferably about 10 to 600 mg, more preferably about 10 to 300 mg, and particularly preferably about 15 to 150 mg in terms of active ingredient (compound represented by formula (I)) per adult (body weight 50 kg), and it is administered in a single dose or in 2 to 3 divided doses per day. In addition, in this case, the pharmaceutical composition may be used in combination with other inhibitors for graft-versus-host disease and/or rejection reaction at the time of organ transplantation. Specific examples of the inhibitors for graft-versus-host disease and/or rejection reaction at the time of organ transplantation used in combination with the compound represented by formula (I) above or a salt thereof include cyclosporine, tacrolimus, rapamycin, steroids, azathioprine, mycophenolate mofetil, mizoribine, and the like. When these drugs are used in combination, if one of the drugs has an influence on metabolism of another drug, then the dosages of the respective drugs are to be adjusted appropriately, but in general, the dosage in the single administration of each drug is used.

When the compound represented by formula (I) above or a salt thereof is used for object diseases other than inhibitors for graft-versus-host disease and/or rejection reaction in cases of organ transplantation, the daily dosage will vary depending on the kind of disease, the patient condition and body weight, and the administration method, but for oral administration, it is about 5 to 1000 mg, preferably with about 10 to 600 mg, more preferably about 10 to 300 mg, and particularly preferably about 15 to 150 mg in terms of active ingredient (compound represented by formula (I)) per adult (body weight 50 kg), and it is administered in a single dose or in 2 to 3 divided doses per day. In addition, when used in combination with other drugs, the dosage of the other drugs is to be selected appropriately within, for example, the range of from about 1/200 to 1/2 or more, to about 2 to 3 times or less of the normal dosage. In addition, when 2 or more drugs are used in combination, if one of the drugs has an influence on metabolism of another drug, then the dosages of the respective drugs are to be adjusted appropriately, but in general, the dosage in the single administration of each drug is used.

In addition, the compound represented by formula (I) above or a salt thereof can be contained in, or used in combination with, blood for transfusion or a blood preparation. Although blood for transfusion or a blood preparation is normally manufactured by mixing blood taken from multiple individuals, there is a case where cells that are not infected and cells that are infected with HIV virus are mixed, and in this case, there is the danger of infection in cells that have not been infected. By blending the compound represented by formula (I) of the present invention, it is possible to prevent or inhibit these viral infection and propagation. In particular, when storing a blood preparation, blending the compound represented by formula (I) is effective for preventing or inhibiting viral infection and propagation. In addition, when blood for transfusion or a blood preparation in which HIV virus is admixed has been administered, by blending the compound represented by formula (I) therein, it is possible to prevent HIV infection and propagation in the individual who was administered the blood for transfusion or blood preparation. For example, when administered orally to adults (body weight about 60 kg) in order to prevent HIV infection during transfusion or during use of a blood preparation, the single dose is normally about 0.02 to 50 mg/kg, preferably 0.05 to 30 mg/kg, and more preferably about 0.1 to 10 mg/kg in terms of CCR antagonist, and it is preferably administered from about 1 to 3 times per day.

Of course, the dosage range may be adjusted based on a unit required to divide the daily dose, but as stated above, the dose is determined in consideration of the properties and extent of the disease; the age, body weight, general physical

condition, and sex of the patient; food; administration time; administration method; excretion rate; and other factors. The administration method may also be selected appropriately in this case, and the above HIV infection preventative agent of the present invention may be added directly to blood for transfusion or blood preparation prior to transfusion or prior to the use of the blood preparation. In such case, it is desirable to mix the agent immediately before to 24 hrs before, preferably immediately before to 12 hrs before, and more preferably immediately before to 6 hrs before the transfusion or use of the blood preparation.

When the HIV infection preventive agent of the present invention is to be administered separately from the blood to be transfused or blood preparation at the time of transfusion or use of the blood preparation, it is preferable to administer 1 hr before the transfusion or use of the blood preparation to simultaneously, and it is more preferable to continue the administration of 1 to 3 times per day for 4 weeks.

In addition, when the compound represented by formula (I) or a salt thereof is used in combination with a reverse transcriptase inhibitor and/or protease inhibitor, the dosage of the reverse transcriptase inhibitor or protease inhibitor, for example, is selected appropriately with a range of from about 1/200 to 1/2 or more to about 2 to 3 times or less relative to the ordinary dosage.

Examples of ordinary dosages for typical reverse transcriptase inhibitors and protease inhibitors are shown below.

Zidovudine: 100 mg
Didanosine: 125-200 mg
Zalcitabine: 0.75 mg
Lamivudine: 150 mg
Stavudine: 30-40 mg
Saquinavir: 600 mg
Ritonavir: 600 mg
Indinavir: 800 mg
Nelfinavir: 750 mg

In addition, specific embodiments are shown below in which the compound represented by formula (I) or a salt thereof is used in combination with a reverse transcriptase inhibitor and/or protease inhibitor.

(a) About 10 to 300 mg of the compound represented by formula (I) or a salt thereof per an adult (body weight 50 kg) is administered in a form of combined use with about 50 to 200 mg of zidovudine to the same subject. Each of the drugs may be administered simultaneously, or may be administered at different times within a 12-hour period.
(b) About 10 to 300 mg of the compound represented by formula (I) or a salt thereof per an adult (body weight 50 kg) is administered to an adult individual (body weight 50 kg) in a form of combined use with about 300 to 1200 mg of saquinavir to the same subject. Each of the drugs may be administered simultaneously, or may be administered at different times within a 12-hour period.

Hereinafter, the present invention will be described further in detail based on Examples, Reference Examples, and Test Examples, but the present invention is not restricted to these Examples.

## Example 1

1 g of (S)-(-)-8-[4-(2-butoxyethoxy)phenyl]-1-isobutyl-N-[4-[[(1-propyl-1H-imidazol-5-yl)methyl]sulfinyl]phenyl]-1,2,3,4-tetrahydro-1-benzazocine-5-carboxamide methanesulfonate (compound A) was dispersed under heating at 60°C in 3.4 g of polyoxyethylene(40)-hydrogenated castor oil, 3.4 g of polyethylene glycol(8)-caprylic acid/capric acid glycerides, and 1.7 g of medium-chain fatty acid triglycerides. Then, 0.5 g of purified water was added to this dispersion liquid, and warmed to obtain a transparent composition solution. The theoretical component ratios are as in Table 1.

[Table 1]

| Component ratio | (-) |
|---|---|
| Compound A | 1.0 |
| Polyoxyethylene(40)-hydrogenated castor oil | 3.4 |
| Polyethylene glycol(8)-caprylic acid/capric acid glycerides | 3.4 |
| Medium-chain fatty acid triglycerides | 1.7 |
| Purified water | 0.5 |

Example 2

1 g of compound A was dispersed under heating at 60°C in 3.4 g of polyoxyethylene(60)-hydrogenated castor oil, 3.4 g of polyethylene glycol(8)-caprylic acid/capric acid glycerides, and 1.7 g of medium-chain fatty acid triglycerides. Then, 0.5 g of purified water was added to this dispersion liquid, and warmed to obtain a transparent composition solution. The theoretical component ratios are as in Table 2.

[Table 2]

| Component ratio | (-) |
|---|---|
| Compound A | 1.0 |
| Polyoxyethylene(60)-hydrogenated castor oil | 3.4 |
| Polyethylene glycol(8)-caprylic acid/capric acid glycerides | 3.4 |
| Medium-chain fatty acid triglycerides | 1.7 |
| Purified water | 0.5 |

Example 3

2 g of compound A was dispersed under heating at 60°C in 3.2 g of polyoxyethylene(40)-hydrogenated castor oil, 3.3 g of polyethylene glycol(8)-caprylic acid/capric acid glycerides, and 1.0 g of medium-chain fatty acid triglycerides. Then, 1.0 g of purified water was added to this dispersion liquid, and warmed to obtain a transparent composition solution. The theoretical component ratios are as in Table 3.

[Table 3]

| Component ratio | (-) |
|---|---|
| Compound A | 2.0 |
| Polyoxyethylene(40)-hydrogenated castor oil | 3.2 |
| Polyethylene glycol(8)-caprylic acid/capric acid glycerides | 3.3 |
| Medium-chain fatty acid triglycerides | 1.0 |
| Purified water | 1.0 |

Example 4

500 g of compound A was dispersed under heating at 60°C in 1700 g of polyoxyethylene(40)-hydrogenated castor oil, 1700 g of polyethylene glycol(8)-caprylic acid/capric acid glycerides and 850 g of medium-chain fatty acid triglycerides. Then, 250 g of purified water was added to this dispersion liquid, and warmed to obtain a transparent composition solution. About 4600 of soft gelatin capsules were produced wherein 293 mg of the resulting transparent composition solution was enclosed per capsule. The theoretical composition per capsule is as in Table 4.

[Table 4]

| Composition | (mg) |
|---|---|
| Compound A | 30 |
| Polyoxyethylene(40)-hydrogenated castor oil | 102 |
| Polyethylene glycol(8)-caprylic acid/capric acid glycerides | 102 |
| Medium-chain fatty acid triglycerides | 51 |
| Purified water | 15 |
| Subtotal | 300 |
| Empty soft capsule (transparent) | 180 |

(continued)

| Composition | (mg) |
|---|---|
| Total | 480 |

Example 5

450 g of compound A was dispersed under heating at 60°C in 1530 g of polyoxyethylene(40)-hydrogenated castor oil, 1530 g of polyethylene glycol(8)-caprylic acid/capric acid glycerides, and 765 g of medium-chain fatty acid triglycerides. Then, 225 g of purified water was added to this dispersion liquid, and warmed to obtain a transparent composition solution. About 10,000 of hard gelatin capsules were produced wherein 284 mg of the resulting transparent composition solution was enclosed per capsule. The theoretical composition per capsule is as in Table 5.

[Table 5]

| Composition | (mg) |
|---|---|
| Compound A | 28.4 |
| Polyoxyethylene(40)-hydrogenated castor oil | 96.56 |
| Polyethylene glycol(8)-caprylic acid/capric acid glycerides | 96.56 |
| Medium-chain fatty acid triglycerides | 48.28 |
| Purified water | 14.2 |
| Subtotal | 284 |
| Empty hard capsule (transparent) | 60 |
| Total | 344 |

Example 6

1 g of compound A was dispersed under heating at 60°C in 2.4 g of polyoxyethylene(40)-hydrogenated castor oil, 4.8 g of polyethylene glycol(8)-caprylic acid/capric acid glycerides, and 1.8 g of medium-chain fatty acid triglycerides. The theoretical component ratios are as in Table 6.

[Table 6]

| Component ratio | (-) |
|---|---|
| Compound A | 1.0 |
| Polyoxyethylene(40)-hydrogenated castor oil | 2.4 |
| Polyethylene glycol(8)-caprylic acid/capric acid glycerides | 4.8 |
| Medium-chain fatty acid triglycerides | 1.8 |

Example 7

1 g of compound A was dispersed under heating at 60°C in 3.4 g of polyoxyethylene 40)-hydrogenated castor oil, 3.4 g of polyethylene glycol(8)-caprylic acid/capric acid glycerides, and 2.2 g of medium-chain fatty acid triglycerides. The theoretical component ratios are as in Table 7.

[Table 7]

| Component ratio | (-) |
|---|---|
| Compound A | 1.0 |
| Polyoxyethylene(40)-hydrogenated castor oil | 3.4 |
| Polyethylene glycol(8)-caprylic acid/capric acid glycerides | 3.4 |

(continued)

| Component ratio | (-) |
|---|---|
| Medium-chain fatty acid triglycerides | 2.2 |

Example 8

500 g of compound A was dispersed under heating at 60°C in 990 g of polyoxyethylene(40)-hydrogenated castor oil, 990 g of polyethylene glycol(8)-caprylic acid/capric acid glycerides, and 500 g of medium-chain fatty acid triglycerides. About 15,000 of hard gelatin capsules were produced wherein 170 mg of the resulting composition solution was enclosed per capsule (Size No. 3 capsule). The theoretical composition per capsule is as in Table 8.

[Table 8]

| Composition | (mg) |
|---|---|
| Compound A | 28.5 |
| Polyoxyethylene(40)-hydrogenated castor oil | 56.5 |
| Polyethylene glycol(8)-caprylic acid/capric acid glycerides | 56.5 |
| Medium-chain fatty acid triglycerides | 28.5 |
| Total of contents in capsule | 170 |
| Empty hard capsule (white) | Size No.3 |

Example 9

500 g of compound A was dispersed under heating at 60°C in 990 g of polyoxyethylene(40)-hydrogenated castor oil, 990 g of polyethylene glycol(8)-caprylic acid/capric acid glycerides, and 500 g of medium-chain fatty acid triglycerides. About 7500 of hard gelatin capsules were produced wherein 340 mg of the resulting composition solution was enclosed per capsule (Size No. 2 capsule). The theoretical composition per capsule is as in Table 9.

[Table 9]

| Composition | (mg) |
|---|---|
| Compound A | 57 |
| Polyoxyethylene(40)-hydrogenated castor oil | 113 |
| Polyethylene glycol(8)-caprylic acid/capric acid glycerides | 113 |
| Medium-chain fatty acid triglycerides | 57 |
| Total of contents in capsule | 340 |
| Empty hard capsule (white) | Size No.2 |

Example 10

500 g of compound A was dispersed under heating at 60°C in 990 g of polyoxyethylene(40)-hydrogenated castor oil, 990 g of polyethylene glycol(8)-caprylic acid/capric acid glycerides, and 500 g of medium-chain fatty acid triglycerides. About 3700 of hard gelatin capsules were produced wherein 680 mg of the resulting composition solution was enclosed per capsule (Size No. 0 capsule). The theoretical composition per capsule is as in Table 10.

[Table 10]

| Composition | (mg) |
|---|---|
| Compound A | 114 |
| Polyoxyethylene(40)-hydrogenated castor oil | 226 |

(continued)

| Composition | (mg) |
|---|---|
| Polyethylene glycol(8)-caprylic acid/capric acid glycerides | 226 |
| Medium-chain fatty acid triglycerides | 114 |
| Total of contents in capsule | 680 |
| Empty hard capsule (white) | (Size No.0 |

Reference Example 1

1 g of compound A was dispersed under heating at 60°C in 6.8 g of polyethylene glycol(8)-caprylic acid/capric acid glycerides and 1.7 g of medium-chain fatty acid triglycerides. Then, 0.5 g of purified water was added to this dispersion liquid, and warmed to obtain a transparent composition solution. The theoretical component ratios are as in Table 11.

[Table 11]

| Component ratio | (-) |
|---|---|
| Compound A | 1.0 |
| Polyethylene glycol(8)-caprylic acid/capric acid glycerides | 6.8 |
| Medium-chain fatty acid triglycerides | 1.7 |
| Purified water | 0.5 |

Reference Example 2

1 g of compound A was dispersed under heating at 60°C in 2.4 g of Polysorbate 80 (Tween 80), 4.8 g of polyethylene glycol(8)-caprylic acid/capric acid glycerides, and 1.8 g of medium-chain fatty acid triglycerides. The theoretical component ratios are as in Table 12.

[Table 12]

| Component ratio | (-) |
|---|---|
| Compound A | 1.0 |
| Polysorbate 80 | 2.4 |
| Polyethylene glycol(8)-caprylic acid/capric acid glycerides | 4.8 |
| Medium-chain fatty acid triglycerides | 1.8 |

Test Example 1

0.3 g of the composition of Example 1 was dispersed in 20 mL of various solvent (A: purified water, B: 1 M sodium chloride aqueous solution, C: Japanese Pharmacopoeia no. 2 solution (pH 6.8)) at 20°C or 40°C, and then, the liquid state in 30 minutes and turbidity ratio calculated by the formula below were evaluated. Similar evaluations were carried out for the composition of Reference Example 1. As shown in Figs. 1 and 2, it was confirmed that with the composition of Example 1, turbidity did not occur regardless of the kind of dispersion solvent and regardless of the temperature, and a stable microemulsion was formed. On the other hand, turbidity was observed in the composition of Reference Example 1, and the growth of the emulsion droplets to macro-level was observed.

$$\text{Turbidity ratio} = X/Y$$

wherein,

Y: Absorption at 550 nm when the composition of Example 1 was dispersed in purified water (25°C)
X: Absorption at a wavelength of 550 nm for each sample

Test Example 2

About 0.3 g of the composition of Example 6 or Reference Example 2 was filled into empty soft gelatin capsules. The capsules were then subjected to elution test (paddle method, 100 rpm) in accordance with Japanese Pharmacopoeia using purified water (900 mL) warmed to 37°C. As shown in Figure 3, the composition of Example 6 produced a transparent test solution, and stable microemulsion formation was observed. Turbidity was observed with the composition of Reference Example 2.

Test Example 3

About 0.284 g of the composition of Example 1 or Reference Example 1 was filled into empty hard gelatin capsules. The capsules were administered to fasting beagles. Plasma was collected over time, and the concentration of the compound A therein was quantified by high-performance liquid chromatography.

Industrial Applicability

The pharmaceutical composition of the present invention forms a stable microemulsion, or can maintain this stable microemulsion. In addition, when a preparation containing the pharmaceutical composition of the present invention is administered orally, a stable microemulsion in which microparticles comprising an active ingredient are dispersed is formed or maintained in the digestive tract, and as a result, the absorbability of the active ingredient, particularly a hardly water-soluble active ingredient from the digestive tract is greatly improved, and the bioavailability thereof becomes higher.

## Claims

1. A semisolid or liquid oral pharmaceutical composition comprising
a medicinal compound having a solubility of less than 0.1 mg/mL in water at 25°C and
two or more surfactants having different molecular weights, each of the surfactants having an HLB of 12 or more, one of the surfactants being a $C_{14-20}$ fatty acid glyceride having, as hydrophilic groups, polyoxyethylene chains in which the number of repetitions of ethylene oxide unit is 20 to 500, and
another of the surfactants being a $C_{4-14}$ fatty acid glyceride having, as hydrophilic groups, polyoxyethylene chains in which the number of repetitions of ethylene oxide unit is 2 to 20.

2. The composition according to claim 1, wherein the content of the surfactants is 10 wt% or more.

3. The composition according to claim 1, wherein the one of the surfactants is polyoxyethylene-hydrogenated castor oil and the other of the surfactants is polyethylene glycol-caprylic acid/capric acid glyceride.

4. The composition according to claim 3, wherein the one of the surfactants is polyoxyethylene(40)-hydrogenated castor oil and the other of the surfactants is polyethylene glycol(8)-caprylic acid/capric acid glyceride.

5. The composition according to claim 1, wherein the blending ratio of the one of the surfactants and the other of the surfactants is 1:10 to 10:1.

6. The composition according to claim 1, wherein the medicinal compound is a compound represented by formula (I) :

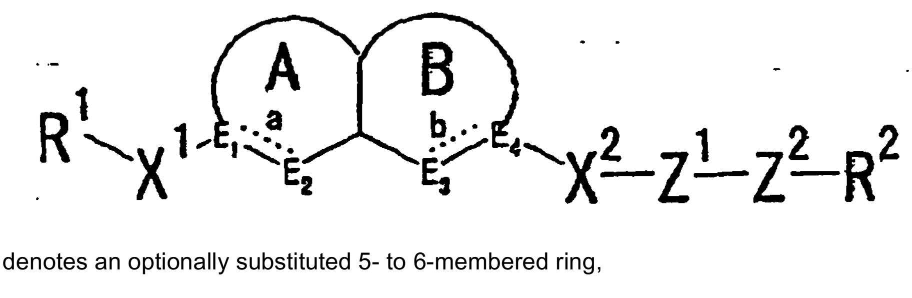

wherein, $R^1$ denotes an optionally substituted 5- to 6-membered ring,

$X^1$ denotes a bond or a divalent group wherein the number of atoms constituting the straight-chain moiety is 1 to 4, ring A denotes an optionally substituted 5- or 6-membered ring,
and ring B denotes an optionally substituted 8- to 10-membered ring,
$E_1$ and $E_4$ each denote an optionally substituted carbon atom or an optionally substituated nitrogen atom,
$E_2$ and $E_3$ each denote an optionally substituted carbon atom, optionally substituted nitrogen atom, optionally oxidized sulfur atom or oxygen atom,
a and b each denote a single bond or a double bond,
$X^2$ denotes a divalent group wherein the number of atoms constituting the straight chain moiety is 1 to 4,
$Z^1$ denotes a bond or a divalent cyclic group,
$Z^2$ denotes a bond or a divalent group,
$R^2$ denotes (1) an optionally substituted amino group whose nitrogen atom may be converted into a quaternary ammonium or oxide, (2) an optionally substituted nitrogen-containing heterocyclic group which may comprise sulfur atoms or oxygen atoms as ring constituent atoms and whose nitrogen atom may be converted into a quaternary ammonium or oxide, (3) a group represented by the formula:

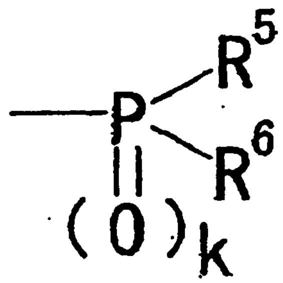

wherein, k denotes 0 or 1, and when k is 0, the phosphorus atom can form a phosphonium salt; $R^5$ and $R^6$ each denote an optionally substituted hydrocarbon group, optionally substituted hydroxyl group, or optionally substituted amino group; and $R^5$ and $R^6$ can be bonded together to form a cyclic group along with the adjacent phosphorus atom, (4) an optionally substituted amidino group, or (5) an optionally substituted guanidine group; or a salt thereof.

**7.** The composition of claim 1, wherein the medicinal compound is selected from
8-[4-(2-butoxyethoxy)phenyl]-N-[4-[[N-methyl-N-(tetrahydropyran-4-yl)amino]methyl)phenyl]-3,4-dihydro-2H-1-benzoxocin-5-carboxamide:
8-[4-(2-butoxyethoxy)phenyl]-N-[4-[[N-methyl-N-(tetrahydropyran-4-yl)amino]methyl]phenyl]-1,2,3,4-tetrahydro-1-benzazocine-5-carboxamide;
8-[4-(2-butoxyethoxy)phenyl]-1-propyl-N-[4-[[[1-propylimidazol-5-yl]methyl]sulfanyl]phenyl]-1,2,3,4-tetrahydro-1-benzazocine-5-carboxamide;
8-[4-(2-butoxyethoxy)phenyl]-1-propyl-N-[4-[[[1-propylimidazol-5-yl]methyl]sulfinyl]phenyl]-1,2,3,4-tetrahydro-1-benzazocine-5-carboxamide;
8-[4-(2-butoxyethoxy)phenyl]-1-propyl-N-[4-[[[1-propylimidazol-5-yl]methyl]sulfonyl]phenyl]-1,2,3,4-tetrahydro-1-benzazocine-5-carboxamide;
8-[4-(2-butoxyethoxy)phenyl]-1-isobutyl-N-[4-[[[1-propylimidazol-5-yl]methyl]sulfanyl]phenyl]-1,2,3,4-tetrahydro-1-benzazocine-5-carboxamide;
8-[4-(2-butoxyethoxy)phenyl]-1-isobutyl-N-[4-[[[1-propylimidazol-5-yl]methyl]sulfinyl]phenyl]-1,2,3,4-tetrahydro-1-benzazocine-5-carboxamide;
8-[4-(2-butoxyethoxy)phenyl]-1-isobutyl-N-[4-[[[1-propylimidazol-5-yl]methyl]sulfonyl]phenyl]-1,2,3,4-tetrahydro-1-benzazocine-5-carboxamide;
8-[4-(2-butoxyethoxy)phenyl]-1-isobutyl-N-[4-[[N-methyl-N-(tetrahydropyran-4-yl)amino]methyl]phenyl]-1,2,3,4-tetrahydro-1-benzazocine-5-carboxamide;
(S)-8-[4-(2-butoxyethoxy)phenyl]-1-isobutyl-N-[4-[[(1-propyl-1H-imidazol-5-yl)methyl]sulfinyl]phenyl]-1,2,3,4-tetrahydro-1-benzazocine-5-carboxamide methanesulfonate;
(S)-8-[4-(2-butoxyethoxy)phenyl]-1-propyl-N-[4-[[(1-propyl-1H-imidazol-5-yl)methyl]sulfinyl]phenyl]-1,2,3,4-tetrahydro-1-benzazocine-5-carboxamide methanesulfonate;
(S)-1-isobutyl-8-[4-(2-propoxyethoxy)phenyl]-N-[4-[[(1-propyl-1H-imidazol-5-yl)metyl]sulfinyl]phenyl]-1,2,3,4-tetrahydro-1-benzazocine-5-carboxamide;
(S)-8-[4-(2-butoxyethoxy)phenyl]-1-[(1-methyl-1H-pyrazol-4-yl)methyl]-N-[4-[[(1-propyl-1H-imidazol-5-yl)methyl]sulfinyl]phenyl]-1,2,3,4-tetrahydro-1-benzazocine-5-carboxamide; and

(S)-8-[4-(2-butoxyethoxy)phenyl]-1-isobutyl-N-[4-[[(4-propyl-4H-1,2,4-triazol-3-yl)methyl]sulfinyl]phenyl]-1,2,3,4-tetrahydro-1-benzazocine-5-carboxamide.

**8.** The composition according to any one of claims 1 to 7 in the form of an emulsion.

**9.** The composition of claim 8 in the form of a micro emulsion.

**10.** A preparation which comprises enclosing the composition according to any one of claims 1 to 9.

**11.** The preparation according to claim 10 in the form of a capsule.

**Patentansprüche**

**1.** Halbfeste oder flüssige orale pharmazeutische Zusammensetzung, umfassend:

eine medizinische Verbindung mit einer Löslichkeit von weniger als 0,1 mg/ml in Wasser bei 25 °C; und
zwei oder mehr Tenside mit unterschiedlichen Molekulargewichten, wobei jedes der Tenside einen HLB-Wert von 12 oder mehr aufweist;
wobei eines der Tenside ein $C_{14}$-$_{20}$-Fettsäureglycerid ist, das als hydrophile Gruppen Polyoxyethylenketten aufweist, bei denen die Anzahl der Wiederholungen der Ethylenoxid-Einheit 20 bis 500 beträgt; und
wobei ein anderes der Tenside ein $C_{4-14}$-Fettsäureglycerid ist, das als hydrophile Gruppen Polyoxyethylenketten aufweist, bei denen die Anzahl der Wiederholungen der Ethylenoxid-Einheit 2 bis 20 beträgt.

**2.** Zusammensetzung gemäß Anspruch 1, wobei der Gehalt an den Tensiden 10 Gew.-% oder mehr beträgt.

**3.** Zusammensetzung gemäß Anspruch 1, wobei es sich bei dem einen der Tenside um Polyoxyethylen-hydriertes-Ricinusöl und bei dem anderen der Tenside um Polyethylenglycol-Caprylsäure/Caprinsäureglycerid handelt.

**4.** Zusammensetzung gemäß Anspruch 3, wobei es sich bei dem einen der Tenside um Polyoxyethylen(40)-hydriertes-Ricinusöl und bei dem anderen der Tenside um Polyethylenglycol(8)-Caprylsäure/Caprinsäureglycerid handelt.

**5.** Zusammensetzung gemäß Anspruch 1, wobei das Mischungsverhältnis des einen der Tenside zu dem anderen der Tenside 1: 10 bis 10: 1 beträgt.

**6.** Zusammensetzung gemäß Anspruch 1, wobei die medizinische Verbindung eine Verbindung ist, die durch Formel (I) dargestellt wird:

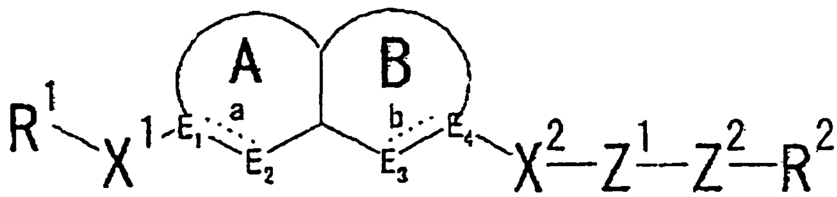

wobei $R^1$ einen gegebenenfalls substituierten fünf- bis sechsgliedrigen Ring bezeichnet;
$X^1$ eine Bindung oder eine zweiwertige Gruppe bezeichnet, wobei die Anzahl der Atome, die die geradkettige Struktureinheit bilden, 1 bis 4 beträgt;
Ring A einen gegebenenfalls substituierten fünf- oder sechsgliedrigen Ring bezeichnet;
und Ring B einen gegebenenfalls substituierten acht- bis zehngliedrigen Ring bezeichnet;
$E_1$ und $E_4$ jeweils ein gegebenenfalls substituiertes Kohlenstoffatom oder ein gegebenenfalls substituiertes Stickstoffatom bezeichnen;
$E_2$ und $E_3$ jeweils ein gegebenenfalls substituiertes Kohlenstoffatom, ein gegebenenfalls substituiertes Stickstoffatom, ein gegebenenfalls oxidiertes Schwefelatom oder ein Sauerstoffatom bezeichnen;
a und b jeweils eine Einfachbindung oder eine Doppelbindung bezeichnen;
$X^2$ eine zweiwertige Gruppe bezeichnet, wobei die Anzahl der Atome, die die geradkettige Struktureinheit bilden, 1 bis 4 beträgt;

$Z^1$ eine Bindung oder eine zweiwertige cyclische Gruppe bezeichnet;
$Z^2$ eine Bindung oder eine zweiwertige Gruppe bezeichnet;
$R^2$ Folgendes bezeichnet: (1) eine gegebenenfalls substituierte Aminogruppe, deren Stickstoffatom in ein quartäres Ammonium oder ein Oxid umgewandelt sein kann, (2) eine gegebenenfalls substituierte stickstoffhaltige heterocyclische Gruppe, die Schwefelatome oder Sauerstoffatome als ringbildende Atome umfassen kann und deren Stickstoffatom in ein quartäres Ammonium oder ein Oxid umgewandelt sein kann, (3) eine Gruppe, die durch die Formel

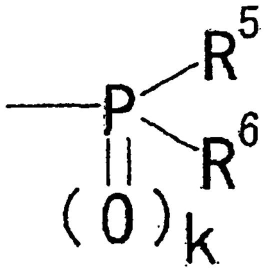

dargestellt wird, wobei k = 0 oder 1 ist und, wenn k = 0 ist, das Phosphoratom ein Phosphoniurnsalz bilden kann, $R^5$ und $R^6$ jeweils eine gegebenenfalls substituierte Kohlenwasserstoffgruppe, gegebenenfalls substituierte Hydroxygruppe oder gegebenenfalls substituierte Aminogruppe bezeichnen und $R^5$ und $R^6$ unter Bildung einer cyclischen Gruppe zusammen mit dem benachbarten Phosphoratom miteinander verbunden sein können, (4) eine gegebenenfalls substituierte Amidinogruppe oder (5) eine gegebenenfalls substituierte Guanidingruppe; oder ein Salz davon ist.

**7.** Zusammensetzung gemäß Anspruch 1, wobei die medizinische Verbindung ausgewählt ist aus
8-[4-(2-Butoxyethoxy)phenyl]-N-[4-[[N-methyl-N-(tetrahydropyran-4-yl)-amino]methyl]phenyl]-3,4-dihydro-2H-1-benzoxocin-5-carboxamid;
8-[4-(2-Butoxyethoxy)phenyl]-N-[4-[[N-methyl-N-(tetrahydropyran-4-yl)-amino]methyl]phenyl]-1,2,3,4-tetrahydro-1-benzazocin-5-carboxamid;
8-[4-(2-Butoxyethoxy)phenyl]-1-propyl-N-[4-[[[1-propylimidazol-5-yl]-methyl]sulfanyl]phenyl]-1,2,3,4-tetrahydro-1-benzazocin-5-carboxamid;
8-[4-(2-Butoxyethoxy)phenyl]-1-propyl-N-[4-[[[1-propylimidazol-5-yl]-methyl]sulfinyl]phenyl]-1,2,3,4-tetrahydro-1-benzazocin-5-carboxamid;
8-[4-(2-Butoxyethoxy)phenyl]-1-propyl-N-[4-[[[1-propylimidazol-5-yl]-methyl]sulfonyl]phenyl]-1,2,3,4-tetrahydro-1-benzazocin-5-carboxamid;
8-[4-(2-Butoxyethoxy)phenyl]-1-isobutyl-N-[4-[[[1-propylimidazol-5-yl]-methyl]sulfanyl]phenyl]-1,2,3,4-tetrahydro-1-benzazocin-5-carboxamid;
8-[4-(2-Butoxyethoxy)phenyl]-1-isobutyl-N-[4-[[[1-propylimidazol-5-yl]-methyl]sulfinyl]phenyl]-1,2,3,4-tetrahydro-1-benzazocin-5-carboxamid;
8-[4-(2-Butoxyethoxy)phenyl]-1-isobutyl-N-[4-[[[1-propylimidazol-5-yl]-methyl]sulfonyl]phenyl]-1,2,3,4-tetrahydro-1-benzazocin-5-carboxamid;
8-[4-(2-Butoxyethoxy)phenyl]-1-isobutyl-N-[4-[[N-methyl-N-(tetrahydropyran-4-yl)amino]methyl]phenyl]-1,2,3,4-tetrahydro-1-benzazocin-5-carboxamid;
(S)-8-[4-(2-Butoxyethoxy)phenyl]-1-isobutyl-N-[4-[[(1-propyl-1H-imidazol-5-yl)methyl]sulfinyl]phenyl]-1,2,3,4-tetrahydro-1-benzazocin-5-carboxamidmethansulfonat;
(S)-8-[4-(2-Butoxyethoxy)phenyl]-1-propyl-N-[4-[[(1-propyl-1H-imidazol-5-yl)methyl]sulfinyl]phenyl]-1,2,3,4-tetrahydro-1-benzazocin-5-carboxamidmethansulfonat;
(S)-1-Isobutyl-8-[4-(2-propoxyethoxy)phenyl]-N-[4-[[(1-propyl-1H-imidazol-5-yl)methyl]sulfinyl]phenyl]-1,2,3,4-tetrahydro-1-benzazocin-5-carboxamid;
(S)-8-[4-(2-Butoxyethoxy)phenyl]-1-[(1-methyl-1H-pyrazol-4-yl)methyl]-N-[4-[[(1-propyl-1H-imidazol-5-yl)methyl] sulfinyl]phenyl]-1,2,3,4-tetra-hydro-1-benzazocin-5-carboxamid; und
(S)-8-[4-(2-Butoxyethoxy)phenyl]-1-isobutyl-N-[4-[[(4-propyl-4H-1,2,4-triazol-3-yl)methyl]sulfinyl]phenyl]-1,2,3,4-tetrahydro-1-benzazocin-5-carboxamid.

**8.** Zusammensetzung gemäß einem der Ansprüche 1 bis 7 in Form einer Emulsion.

**9.** Zusammensetzung gemäß Anspruch 8 in Form einer Mikroemulsion.

**10.** Präparat, das das Einschließen der Zusammensetzung gemäß einem der Ansprüche 1 bis 9 umfasst.

**11.** Präparat gemäß Anspruch 10 in Form einer Kapsel.

**Revendications**

**1.** Composition pharmaceutique orale semi-solide ou liquide comprenant
un médicament présentant une solubilité inférieure à 0,1 mg/ml dans l'eau à 25 °C et
deux surfactants ou plus présentant des masses moléculaires différentes, chacun des surfactants présentant un HLB de 12 ou plus,
l'un des surfactants étant un glycéride d'acide gras en $C_{14}$ à $C_{20}$ comportant, en tant que groupes hydrophiles, des chaînes de polyoxyéthylène dans lesquelles le nombre de répétitions d'unité d'oxyde d'éthylène est de 20 à 500, et un autre des surfactants étant un glycéride d'acide gras en $C_4$ à $C_{14}$ comportant, en tant que groupes hydrophiles, des chaînes de polyoxyéthylène dans lesquelles le nombre de répétitions d'unité d'oxyde d'éthylène est de 2 à 20.

**2.** Composition selon la revendication 1, dans laquelle la teneur des surfactants est de 10 % en poids ou plus.

**3.** Composition selon la revendication 1, dans laquelle l'un des surfactants est de l'huile de ricin hydrogénée polyoxyéthylénée et l'autre des surfactants est du polyéthylène glycol-glycéride d'acide caprylique/d'acide caprique.

**4.** Composition selon la revendication 3, dans laquelle l'un des surfactants est de l'huile de ricin hydrogénée polyoxyéthylénée (40) et l'autre des surfactants est du polyéthylène glycol (8)-glycéride d'acide caprylique/d'acide caprique.

**5.** Composition selon la revendication 1, dans laquelle le rapport de mélange de l'un des surfactants et de l'autre des surfactants est de 1/10 à 10/1.

**6.** Composition selon la revendication 1, dans laquelle le médicament est un composé représenté par la formule (I) :

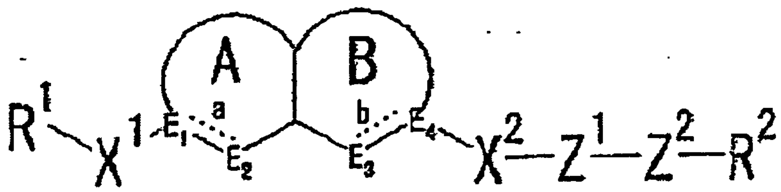

dans laquelle, $R^1$ indique un cycle de 5 ou 6 chaînons éventuellement substitué,
$X^1$ indique une liaison ou un groupe bivalent où le nombre d'atomes constituant le radical de chaîne linéaire est de 1 à 4,
le cycle A indique un cycle de 5 ou 6 chaînons éventuellement substitué,
et le cycle B indique un cycle de 8 à 10 chaînons éventuellement substitué,
$E_1$ et $E_4$ indiquent chacun un atome de carbone éventuellement substitué ou un atome d'azote éventuellement substitué,
$E_2$ et $E_3$ indiquent chacun un atome de carbone éventuellement substitué, un atome d'azote éventuellement substitué, un atome de soufre éventuellement oxydé ou un atome d'oxygène,
a et b indiquent chacun une liaison simple ou une double liaison,
$X^2$ indique un groupe bivalent où le nombre d'atomes constituant le radical de la chaîne linéaire est de 1 à 4,
$Z^1$ indique une liaison ou un groupe cyclique bivalent,
$Z^2$ indique une liaison ou un groupe bivalent,
$R^2$ indique (1) un groupe amino éventuellement substitué dont l'atome d'azote peut être converti en un ammonium quaternaire ou un oxyde, (2) un groupe hétérocyclique contenant de l'azote éventuellement substitué qui peut comprendre des atomes de soufre ou des atomes d'oxygène en tant qu'atomes constituants du cycle et dont l'atome d'azote peut être converti en un ammonium quaternaire ou un oxyde, (3) un groupe représenté par la formule :

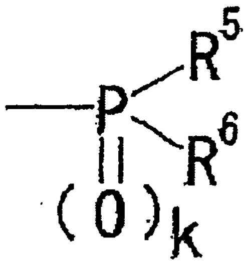

dans laquelle, k indique 0 ou 1, et lorsque k vaut 0, l'atome de phosphore peut former un sel de phosphonium ; $R^5$ et $R^6$ indiquent chacun un groupe d'hydrocarbure éventuellement substitué, un groupe hydroxyle éventuellement substitué, ou un groupe amino éventuellement substitué ; et $R^5$ et $R^6$ peuvent être liés ensemble pour former un groupe cyclique accompagné de l'atome de phosphore adjacent, (4) un groupe amidino éventuellement substitué, ou (5) un groupe guanidine éventuellement substitué ; ou un sel de celui-ci.

**7.** Composition selon la revendication 1, dans laquelle le médicament est choisi parmi
le 8-[4-(2-butoxyéthoxy)phényl]-N-[4-[[N-méthyl-N-(tétrahydropyran-4-yl)amino]méthyl]phényl]-3,4-dihydro-2H-1-benzoxocine-5-carboxamide ;
le 8-[4-(2-butoxyéthoxy)phényl]-N-[4-[[N-méthyl-N-(tétrahydropyran-4-yl)amino]méthyl]phényl]-1,2,3,4-tétrahydro-1-benzazocine-5-carboxamide ;
le 8-[4-(2-butoxyéthoxy)phényl]-1-propyl-N-[4-[[[1-propylimidazol-5-yl]méthyl]sulfanyl]phényl]-1,2,3,4-tétrahydro-1-benzazocine-5-carboxamide ;
le 8-[4-(2-butoxyéthoxy)phényl]-1-propyl-N-[4-[[[1-propylimidazol-5-yl]méthyl]sulfinyl]phényl]-1,2,3,4-tétrahydro-1-benzazocine-5-carboxamide ;
le 8-[4-(2-butoxyéthoxy)phényl]-1-propyl-N-[4-[[[1-propylimidazol-5-yl]méthyl]sulfonyl]phényl]-1,2,3,4-tétrahydro-1-benzazocine-5-carboxamide ;
le 8-[4-(2-butoxyéthoxy)phényl]-1-isobutyl-N-[4-[[[1-propylimidazol-5-yl]méthyl]sulfanyl]phényl]-1,2,3,4-tétrahydro-1-benzazocine-5-carboxamide ;
le 8-[4-(2-butoxyéthoxy)phényl]-1-isobutyl-N-[4-[[[1-propylimidazol-5-yl]méthyl]sulfinyl]phényl]-1,2,3,4-tétrahydro-1-benzazocine-5-carboxamide ;
le 8-[4-(2-butoxyéthoxy)phényl]-1-isobutyl-N-[4-[[[1-propylimidazol-5-yl]méthyl]sulfonyl]phényl]-1,2,3,4-tétrahydro-1-benzazocine-5-carboxamide ;
le 8-[4-(2-butoxyéthoxy)phényl]-1-isobutyl-N-[4-[[N-méthyl-N-(tétrahydropyran-4-yl)amino]méthyl]-phényl]-1,2,3,4-tétrahydro-1-benzazocine-5-carboxamide ;
le méthanesulfonate de (S)-8-[4-(2-butoxyéthoxy)-phényl]-1-isobutyl-N-[4-[[(1-propyl-1H-imidazol-5-yl)-méthyl]sulfinyl]phényl]-1,2,3,4-tétrahydro-1-benzazocine-5-carboxamide ;
le méthanesulfonate de (S)-8-[4-(2-butoxyéthoxy)-phényl]-1-propyl-N-[4-[[(1-propyl-1H-imidazol-5-yl)-méthyl]sulfinyl]phényl]-1,2,3,4-tétrahydro-1-benzazocine-5-carboxamide ;
le (S)-1-isobutyl-8-[4-(2-propoxyéthoxy)phényl]-N-[4-[[(1-propyl-1H-imidazol-5-yl)méthyl]sulfinyl]-phényl]-1,2,3,4-tétrahydro-1-benzazocine-5-carboxamide ;
le (S)-8-[4-(2-butoxyéthoxy)phényl]-1-[(1-méthyl-1H-pyrazol-4-yl)méthyl]-N-[4-[[(1-propyl-1H-imidazol-5-yl)méthyl]sulfinyl]phényl]-1,2,3,4-tétrahydro-1-benzazocine-5-carboxamide ; et
le (S)-8-[4-(2-butoxyéthoxy)phényl]-1-isobutyl-N-[4-[[(4-propyl-4H-1,2,4-triazol-3-yl)méthyl]sulfinyl]-phényl]-1,2,3,4-tétrahydro-1-benzazocine-5-carboxamide.

**8.** Composition selon l'une quelconque des revendications 1 à 7 sous la forme d'une émulsion.

**9.** Composition selon la revendication 8 sous la forme d'une microémulsion.

**10.** Préparation qui comprend l'inclusion de la composition selon l'une quelconque des revendications 1 à 9.

**11.** Préparation selon la revendication 10 sous la forme d'une capsule.

[Figure 1]

Example 1 (20°C)

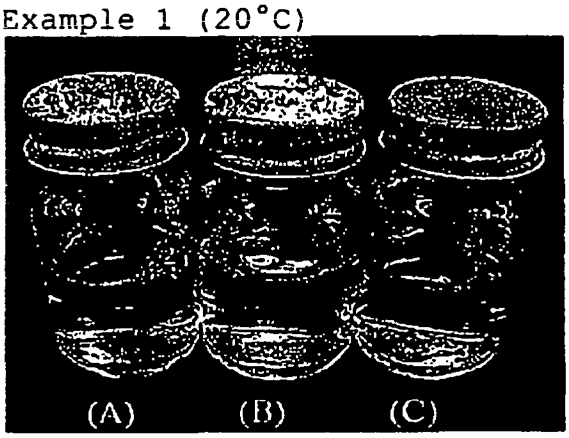


Reference Example 1 (20°C)

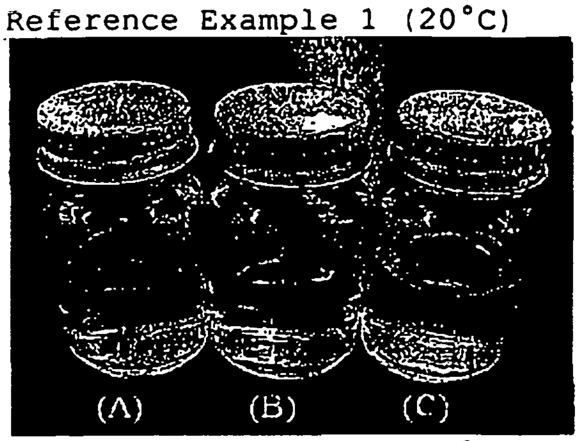


Example 1 (40°C)

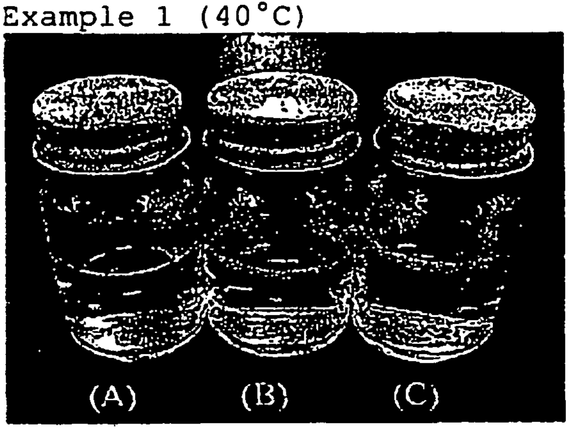


Reference Example 1 (40°C)

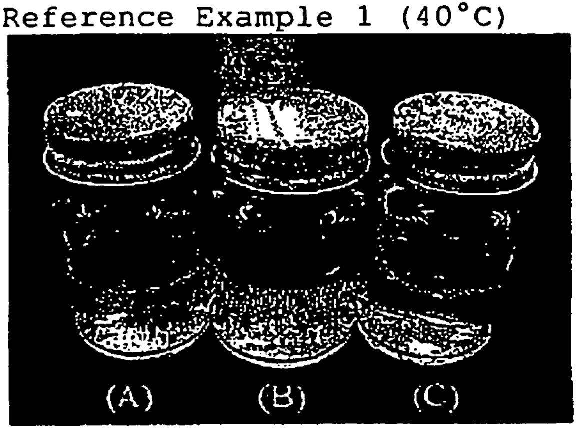

[Figure 2]

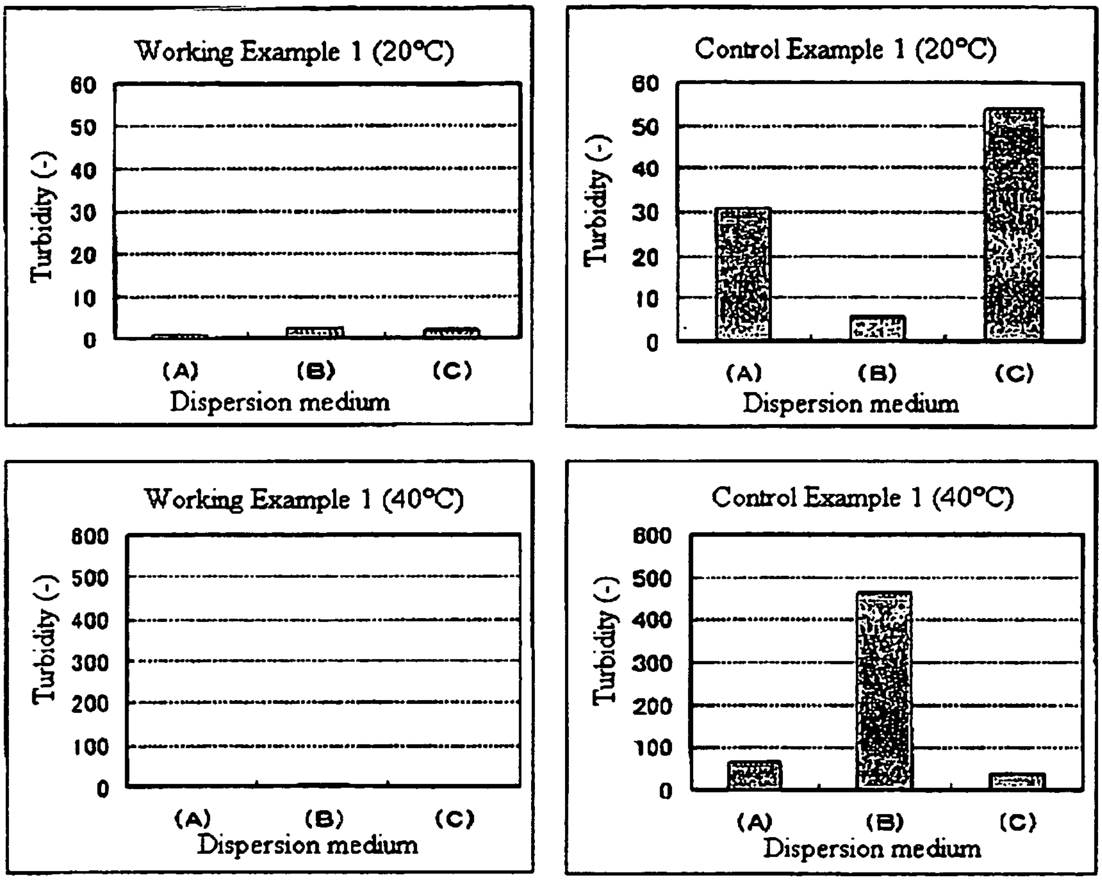
Working Example 1 (20°C)
Turbidity (-)
60
50
40
30
20
10
0
(A)
(B)
(C)
Dispersion medium
Control Example 1 (20°C)
Turbidity (-)
60
50
40
30
20
10
0
(A)
(B)
(C)
Dispersion medium
Working Example 1 (40°C)
Turbidity (-)
600
500
400
300
200
100
0
(A)
(B)
(C)
Dispersion medium
Control Example 1 (40°C)
Turbidity (-)
600
500
400
300
200
100
0
(A)
(B)
(C)
Dispersion medium

[Figure 3]

Example 6

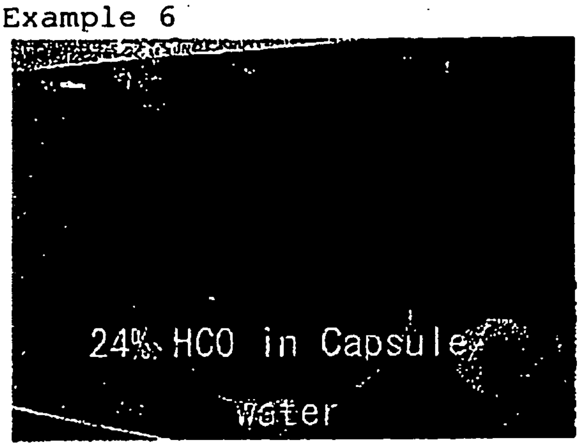


Reference Example 2

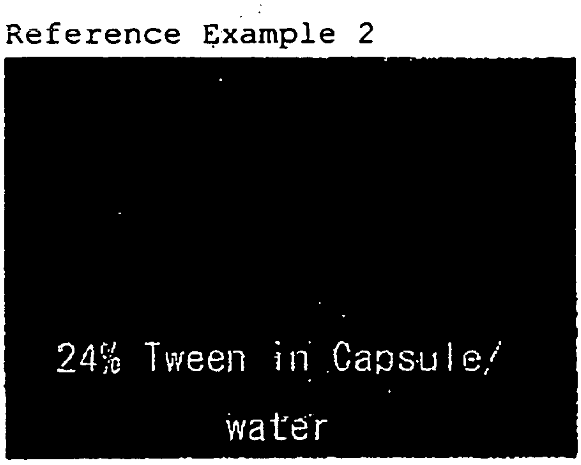


[Figure 4]

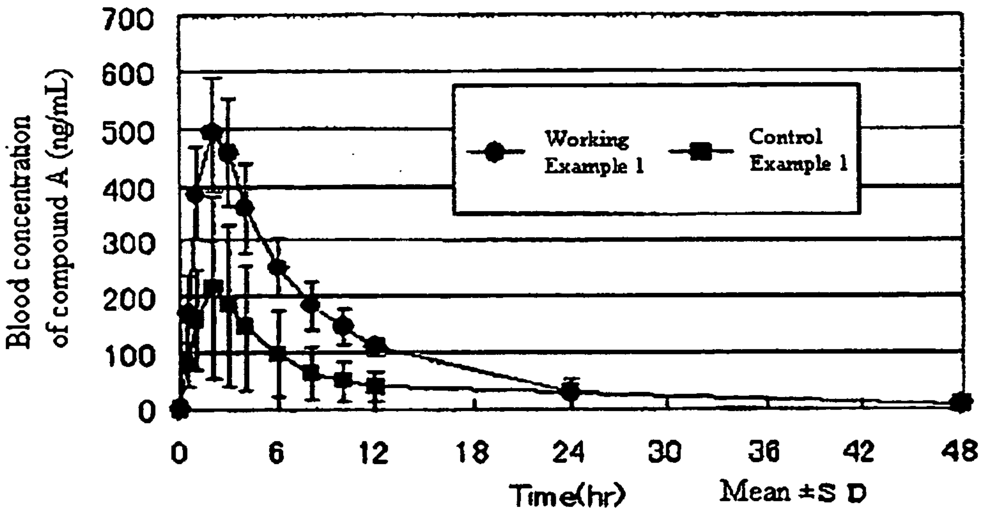

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6054136 A
- WO 03055466 A
- EP 1236476 A
- JP 2003335776 A
- JP 8073476 A